(19) Europäisches Patentamt / European Patent Office / Office européen des brevets

(11) **EP 4 667 979 A1**

(12) **EUROPEAN PATENT APPLICATION**

(43) Date of publication:
24.12.2025 Bulletin 2025/52

(21) Application number: 25179126.5

(22) Date of filing: 27.05.2025

(51) International Patent Classification (IPC):
*G01T 1/36* (2006.01)        *G01T 7/00* (2006.01)

(52) Cooperative Patent Classification (CPC):
G01T 1/36; G01T 7/005

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 19.06.2024 US 202418747809

(71) Applicant: **GE Precision Healthcare LLC Waukesha, WI 53188 (US)**

(72) Inventors:
• **SAXENA, Shefali**
  **10691 Stockholm (SE)**
• **ADAMAK, Mark**
  **Waukesha, 53188 (US)**
• **YANOFF, Brian**
  **Niskayuna, 12309 (US)**

(74) Representative: **AWA Sweden AB**
**Box 45086**
**104 30 Stockholm (SE)**

(54) **SUPPORTING CALIBRATION OF AN X-RAY IMAGING SYSTEM AND ADJUSTMENT OF OPERATIONAL SETTINGS**

(57)    A system and method for supporting calibration of an X-ray imaging system, wherein the X-ray imaging system comprises a multi-bin photon counting X-ray detector having multiple energy bin thresholds. The method includes performing a set of X-ray attenuation measurements or measurement scans of at least one object, using different settings of the energy bin thresholds and obtaining information about material composition related to the object(s). The method further includes determining, for each X-ray attenuation measurement or measurement scan, a value of at least one performance metric related to the X-ray imaging system and selecting a custom set of energy bin thresholds based on the determined values of the performance metrics over the set of X-ray attenuation measurements or measurement scans. Also, the method includes determining calibration data coupling the selected custom set of energy bin thresholds to at least the information about material composition.

Performing a set of X-ray attenuation measurements, using different settings of energy bin thresholds — S1

Obtaining information about material composition — S2

Determining, for each X-ray attenuation measurement, a value of at least one performance metric — S3

Selecting a custom set of energy bin thresholds — S4

Determining calibration data — S5

*FIG. 11*

EP 4 667 979 A1

**Description**

BACKGROUND

The proposed technology relates to X-ray technology and X-ray imaging, and particularly to supporting calibration of an X-ray imaging system. More specifically, the proposed technology relates to a method and system for supporting calibration of an X-ray imaging system having a multi-bin photon counting X-ray detector, and a corresponding Computed Tomography (CT) imaging system and method for adjusting operational settings for an X-ray imaging system, such as a CT imaging system, as well as a corresponding computer-program product, for improved image quality.

Radiographic imaging such as CT imaging systems and other more general X-ray imaging systems have been used for years in medical applications, such as for medical diagnostics and treatment.

A typical X-ray imaging system such as a CT imaging system includes an X-ray source, an X-ray detector, and an associated image processing system. The X-ray detector includes multiple detector modules comprising one or many detector elements, for independent measuring of X-ray intensities. The X-ray source emits X-rays, which pass through a subject or object being imaged and received by the X-ray detector. The X-ray source and X-ray detector are typically arranged to rotate on a rotating member of a gantry, around the subject or object. The emitted X-rays are attenuated by the subject or object as they pass through, and the resulting transmitted X-rays are measured by the X-ray detector. The X-ray detector is coupled to a digital acquisition system (DAS) and the measured X-ray data is transferred to the image processing system to reconstruct images of the subject or object.

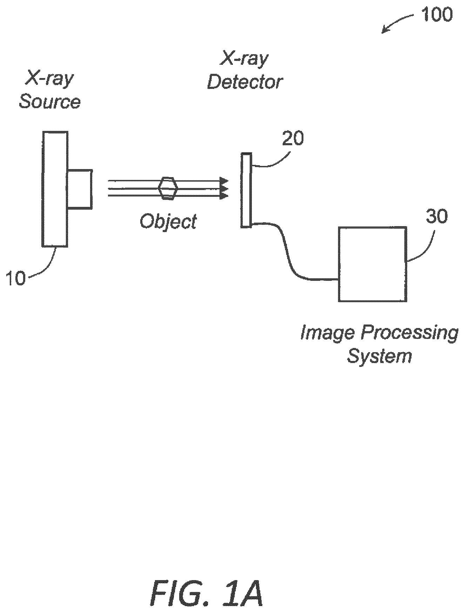

It may be useful with a brief overview of an illustrative general X-ray imaging system according to the prior art with reference to FIG. 1A. In this illustrative example the X-ray imaging system 100 comprises an X-ray source 10, an X-ray detector 20 and an associated image processing system 30. In general, the X-ray detector 20 is configured to register radiation from the X-ray source 10, which optionally has been focused by optional X-ray optics or collimators and passed through an object, a subject or a part thereof. The X-ray detector 20 is connectable to the image processing system 30 via suitable read-out electronics, which is at least partly integrated in the X-ray detector 20, to enable image processing and/or image reconstruction by the image processing system 30.

By way of example, a conventional CT imaging system includes an X-ray source and an X-ray detector arranged in such a way that projection images of the subject or object can be acquired in different viewing angles covering at least 180 degrees. This is most commonly achieved by mounting the source and detector on a support, e.g., a rotating member of a gantry, that is able to rotate around the subject or object. An image containing the projections registered in the different detector elements for the different view angles is called a sinogram. In the following, a collection of projections registered in the different detector elements for different view angles will be referred to as a sinogram even if the detector is two-dimensional (2D), making the sinogram a three-dimensional (3D) image.

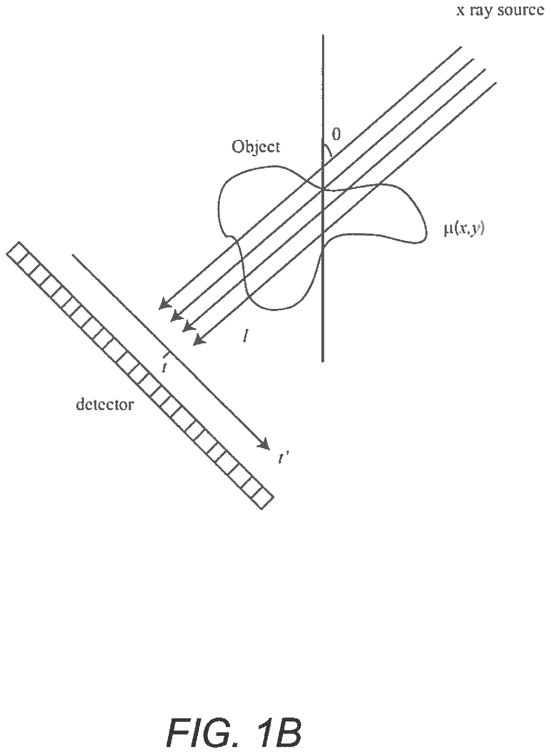

FIG. 1B is a schematic diagram illustrating an example of an X-ray imaging system setup according to the prior art, showing projection lines from an X-ray source through an object to an X-ray detector.

A further development of X-ray imaging is energy-resolved X-ray imaging, also known as spectral X-ray imaging, where the X-ray transmission is measured for several different energy levels. This can be achieved by letting the source switch rapidly between two different emission spectra, by using two or more X-ray sources emitting different X-ray spectra, or by using an energy-discriminating detector which measures the incoming radiation in two or more energy levels. An example of such a detector is a multi-bin photon counting detector, where each registered photon generates a current pulse which is compared to a set of thresholds, thereby counting the number of photons incident in each of a number of energy bins.

A spectral X-ray projection measurement results in a projection image for each energy level. A weighted sum of these projection images can be made to optimize the contrast-to-noise ratio (CNR) for a specified imaging task as described in "SNR and DQE analysis of broad spectrum X-ray imaging", Tapiovaara and Wagner, Phys. Med. Biol. 30, 519.

Another technique enabled by energy-resolved X-ray imaging is basis material decomposition. This technique utilizes the fact that all substances built up from elements with low atomic number, such as human tissue, have linear attenuation coefficients whose energy dependence can be expressed, to a good approximation, as a linear combination of two (or more) basis functions:

$$\mu(E) = a_1 f_1(E) + a_2 f_2(E)$$

where $f_1$ and $f_2$ are basis functions and $a_1$ and $a_2$ are the corresponding basis coefficients. More, generally, $f_i$ are basis functions and $a_i$ are corresponding basis coefficients, where $i = 1, \ldots, N$ where $N$ is the total number of basis functions. If there is one or more element in the imaged volume with high atomic number, high enough for a K-absorption edge to be present in the energy range used for the imaging, one basis function must be added for each such element. In the field of medical imaging, such K-edge elements can typically be iodine or gadolinium, substances that are used as contrast agents.

Basis material decomposition has been described in "Energy-selective reconstructions in X-ray computerized tomography", Alvarez, Macovski, Phys Med Biol. 1976; 21(5):733-744. In basis material decomposition, the integral of each of the basis coefficients, $A_i = \int_\ell a_i dl$ for $i = 1, \ldots, N$ where $N$ is the number of basis functions, is inferred from the measured data in each projection ray $\ell$ from the source to a detector element. In one implementation, this is accomplished by first expressing the expected registered number of counts in each energy bin as a function of $A_i$:

$$\lambda_i = \int_{E=0}^{\infty} S_i(E)\exp\left(-\sum_{j=1}^{N} A_j f_j(E)\right) dE$$

where $\lambda_i$ is the expected number of counts in energy bin $i$, $E$ is the energy, $S_i$ is a response function which depends on the spectrum shape incident on the imaged object, the quantum efficiency of the detector and the sensitivity of energy bin i to X-rays with energy $E$. Even though the term "energy bin" is most commonly used for photon counting detectors, this formula can also describe other energy resolving X-ray imaging systems such as multi-layer detectors, kVp switching X-ray sources or multiple X-ray source systems.

Then, the maximum likelihood method may be used to estimate $A_i$, under the assumption that the number of counts in each bin is a Poisson distributed random variable. This is accomplished by minimizing the negative log-likelihood function, e.g., see "K-edge imaging in X-ray computed tomography using multi-bin photon counting detectors", Roessl and Proksa, Phys. Med. Biol. 52 (2007), 4679-4696:

$$\hat{A}_1, \ldots, \hat{A}_N = \operatorname*{argmin}_{A_1, \ldots, A_N} \sum_{i=1}^{M_b} \lambda_i(A_1, \ldots, A_N) - m_i \ln\lambda_i(A_1, \ldots, A_N)$$

where $m_i$ is the number of measured counts in energy bin $i$ and $M_b$ is the number of energy bins.

When the resulting estimated basis coefficient line integral $\hat{A}_i$ for each projection line is arranged into an image matrix, the result is a material specific projection image, also called a basis image or sinogram, for each basis $i$. This basis image or sinogram can either be viewed directly (e.g., in projection X-ray imaging) or taken as input to a reconstruction algorithm to form maps of basis coefficients $a_i$ inside the object (e.g., in CT imaging). In either case, the result of a basis decomposition can be regarded as one or more basis image or sinogram representations, such as the basis coefficient line integrals or the basis coefficients themselves.

Image quality improvement for X-ray imaging systems is undoubtedly a critical area to ensure quality and safety in patient care, and is associated with various approaches, not to mention within the field of spectral X-ray imaging.

Therefore, there is still a general demand for improvements of image quality in terms of reduced noise, increased contrast to noise ratio (CNR), improved patient does efficiency, etc.

SUMMARY

This summary introduces concepts that are described in more detail in the detailed description. It should not be used to identify essential features of the claimed subject matter, nor to limit the scope of the claimed subject matter.

According to an aspect, there is provided a method for supporting calibration of an X-ray imaging system, wherein the X-ray imaging system comprises a multi-bin photon counting X-ray detector having multiple energy bin thresholds. The method comprises performing a set of X-ray attenuation measurements or measurement scans of at least one object, using different settings of the energy bin thresholds and obtaining information about material composition related to the at least one object. The method further comprises determining, for each X-ray attenuation measurement or measurement scan, a value of at least one performance metric related to the X-ray imaging system and selecting a custom set of energy bin thresholds based on the determined values of the at least one performance metric over the set of X-ray attenuation measurements or measurement scans. Also, the method comprises determining calibration data coupling the selected custom set of energy bin thresholds to at least the information about material composition.

According to an aspect, there is provided a system for supporting calibration of an X-ray imaging system comprising a multi-bin photon counting X-ray detector having multiple energy bin thresholds, wherein the system is configured to perform the aforementioned method.

According to an aspect, there is provided a CT imaging system comprising the aforementioned system for supporting calibration of an X-ray imaging system.

According to an aspect, there is provided a method for adjusting operational settings for an X-ray imaging system comprising a multi-bin photon counting X-ray detector having multiple energy bin thresholds. The method comprises obtaining information representative of material composition related to at least one object and selecting a custom set of energy bin thresholds based on at least the information representative of material composition by using at least the information representative of material composition as input to a look-up data structure or function holding calibration data for retrieval of a set of energy bin thresholds that matches or corresponds to the information representative of material composition. The method further comprises applying the selected custom set of energy bin thresholds as at least part of operational settings of the multi-bin photon counting X-ray detector of the X-ray imaging system.

According to an aspect, there is provided a system for adjusting operational settings for an X-ray imaging system comprising a multi-bin photon counting X-ray detector having multiple energy bin thresholds, wherein the system is configured to perform the aforementioned method.

According to an aspect, there is provided a CT imaging system comprising the aforementioned system for adjusting operational settings for an X-ray imaging system.

According to an aspect, there is provided a computer-program product comprising a non-volatile computer-readable storage medium having stored thereon a computer program, the computer program comprising instructions, which when executed by a processor, cause the processor to perform any of the aforementioned methods.

The proposed technology enables multi-energy bin photon counting detectors having the capacity to improve the diagnostic quality of X-ray images and also realizing that the energy bin thresholds should be carefully selected to improve performance metrics such as the overall contrast to noise ratio. The placement of the energy bin thresholds is important in improving image quality. Therefore, an improved adaptive energy threshold selection system and method is proposed.

BRIEF DESCRIPTION OF DRAWINGS

Various aspects of this disclosure may be better understood upon reference to the accompanying drawings and reading the detailed description.

FIGS. 1A and 1B are schematic diagrams illustrating an example X-ray imaging system.

FIG. 2 is a schematic diagram illustrating another example of an X-ray imaging system, such as a CT imaging system.

FIG. 3 is a schematic block diagram of a CT imaging system as an illustrative example of an X-ray imaging system.

FIG. 4 is a schematic diagram illustrating another example of relevant parts of an X-ray imaging system, such as a CT imaging system.

FIG. 5 is a schematic illustration of a photon counting circuit and/or device according to an exemplary embodiment.

FIG. 6 is a schematic diagram illustrating an example of a semiconductor detector sub-module according to an exemplary embodiment.

FIG. 7 is a schematic diagram illustrating an example of semiconductor detector sub-module according to another exemplary embodiment.

FIG. 8A is a schematic diagram illustrating an example of a semiconductor detector sub-module according to yet another exemplary embodiment.

FIG. 8B is a schematic diagram illustrating an example of a set of tiled detector sub-modules, where each detector sub-module is a depth-segmented detector sub-module and Application Specific Integrated Circuits (ASICs) or corresponding circuitry are arranged below the detector sub-modules as seen from the direction of the incoming X-rays.

FIG. 9 is a schematic diagram illustrating an example of a CT imaging system.

FIG. 10 is a schematic diagram illustrating an example of a design of an X-ray source and X-ray detector system.

FIG. 11 is a schematic flow diagram illustrating a method for supporting calibration of an X-ray imaging system.

FIG. 12 is a schematic flow diagram illustrating a method for supporting calibration of an X-ray imaging system.

FIG. 13 is a schematic diagram illustrating various options for calibration data of an X-ray imaging system according to exemplary embodiments.

FIG. 14 is a schematic flow diagram illustrating a method for adjusting operational settings for an X-ray imaging system.

FIG. 15 is a schematic diagram illustrating an example of look-up data function or structure.

FIG. 16 is a schematic diagram illustrating an example of how to obtain information about material composition based on a scan type.

FIG. 17 is a schematic block diagram illustrating a system for supporting calibration of an X-ray imaging system.

FIG. 18 is a schematic block diagram illustrating a system for adjusting operational settings for an X-ray imaging system.

FIG. 19 is a schematic flow diagram illustrating a method for adjusting operational settings for an X-ray imaging system according to an exemplary embodiment.

FIG. 20 is a schematic diagram illustrating an example of an arrangement of detector sub-modules and an associated arrangement of detector sub-pixels according to an exemplary embodiment.

FIG. 21 is a schematic flow diagram illustrating a method for supporting calibration of an X-ray imaging system according to an exemplary embodiment.

FIG. 22 is a schematic flow diagram illustrating a method for supporting calibration of an X-ray imaging system according to an exemplary embodiment.

FIG. 23 is a table illustrating examples of calibration data for supporting calibration of an X-ray imaging system according to an exemplary embodiment.

FIG. 24 is a graph illustrating examples of a relation between energy (keV) and noise standard deviation of monochromatic attenuation for different energy bin thresholds according to an exemplary embodiment.

FIG. 25 is a schematic flow diagram illustrating a method for adjusting operational settings for an X-ray imaging system according to an exemplary embodiment.

FIG. 26 is a schematic diagram illustrating an example of a computer implementation according to an embodiment.

DETAILED DESCRIPTION

Embodiments of the present disclosure will now be described, by way of example, with reference to the figures.
For a better understanding, it may be useful to continue with an introductory description of non-limiting examples of an overall X-ray imaging system in which data processing and transferring according to the inventive concept may be implemented.

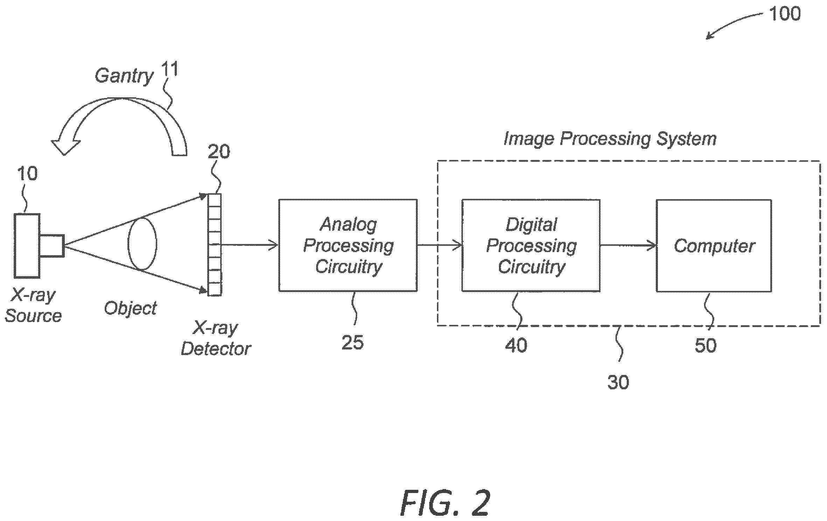

FIG. 2 is a schematic diagram illustrating an example of an X-ray imaging system 100, such as a CT imaging system, comprising an X-ray source 10, which emits X-rays, an X-ray detector 20 with an X-ray detector, which detects X-rays after they have passed through the object, analog processing circuitry 25, which processes the raw electrical signals from the X-ray detector and digitizes it, digital processing circuitry 40, which may carry out further processing operations on the measured data, such as applying corrections, storing it temporarily, or filtering, and a computer 50, which stores the processed data and may perform further post-processing and/or image reconstruction. The digital processing circuitry 40 may comprise a digital processor. According to an exemplary embodiment, all or part of the analog processing circuitry 25 may be implemented in the X-ray detector 20. The X-ray source and X-ray detector may be coupled to a rotating member of a gantry 11 of the CT imaging system 100.
The overall X-ray detector may be regarded as the X-ray detector system 20, or the X-ray detector 20 combined with the associated analog processing circuitry 25.
In communication with and electrically coupled to the analog processing circuitry 25 is an image processing system 30, which may include digital processing circuitry 40 and/or a computer 50, which may be configured to perform image reconstruction based on the image data from the X-ray detector. The image processing system 30 may, thus, be seen as the computer 50, or alternatively the combined system of the digital processing circuitry 40 and the computer 50, or possibly the digital processing circuitry 40 by itself if the digital processing circuitry is further specialized also for image processing and/or reconstruction.

An example of a commonly used X-ray imaging system is a CT imaging system, which may include an X-ray source or X-ray tube that produces a fan beam or cone beam of X-rays and an opposing array of X-ray detectors measuring the fraction of X-rays that are transmitted through a patient or object. The X-ray source or X-ray tube and X-ray detector are mounted in a gantry 11 that can rotate around the imaged object.

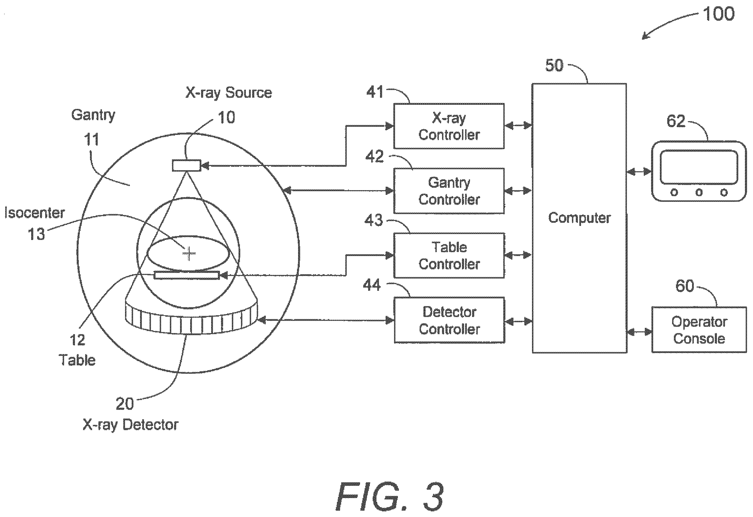

FIG. 3 schematically shows a CT imaging system 100 as an illustrative example of an X-ray imaging system. The CT imaging system comprises a computer 50 receiving commands and scanning parameters from an operator via an operator console 60 that may have a display 62 and some form of operator interface, e.g., a keyboard, mouse, joy stick, touch screen or other input device. The operator supplied commands and parameters are then used by the computer 50 to provide control signals to an X-ray controller 41, a gantry controller 42 and a table controller 43. To be specific, the X-ray controller 41 provides power and timing signals to the x-ray source 10 to control emission of X-rays onto the object or patient lying on the table 12. The gantry controller 42 controls the rotating speed and position of the gantry 11 comprising the X-ray source 10 and the X-ray detector 20. By way of example, the X-ray detector 20 may be a photon counting X-ray detector. The table controller 43 controls and determines the position of the patient table 12 and the scanning coverage of the patient. There is also a detector controller 44, which is configured for controlling and/or receiving data from the X-ray detector 20.

In an embodiment, the computer 50 also performs post-processing and image reconstruction of the image data output from the X-ray detector 20. The computer 50 thereby corresponds to the image processing system 30 as shown in FIGS. 1 and 2. The associated display 62 allows the operator to observe the reconstructed images and other data from the computer 50.

The X-ray source 10 arranged in the gantry 11 emits X-rays. An X-ray detector 20, which may be in the form of a photon counting X-ray detector, detects the X-rays after they have passed through the object or patient. The X-ray detector 20 may for example be formed by plurality of pixels, also referred to as sensors or detector elements, and associated image processing circuitry, such as Application Specific Integrated Circuits (ASICs), arranged in detector modules. A portion of the analog processing may be implemented in the pixels, whereas any remaining processing is implemented in, for instance, the ASICs. In an embodiment, the image processing circuitry (ASICs) digitizes the analog signals from the pixels. The image processing circuitry (ASICs) may also comprise a digital processing, which may carry out further processing operations on the measured data, such as applying corrections, storing it temporarily, and/or filtering. During a scan to acquire X-ray projection data, the gantry and the components mounted thereon rotate about an isocenter 13.

Modern X-ray detectors normally need to convert the incident X-rays into electrons, this typically takes place through the photoelectric effect or through Compton interaction and the resulting electrons are usually creating secondary visible light until its energy is lost and this light is in turn detected by a photo-sensitive material. There are also detectors, which are based on semiconductors and in this case the electrons created by the X-ray are creating electric charge in terms of electron-hole pairs which are collected through an applied electric field.

There are detectors operating in an energy integrating mode in the sense that they provide an integrated signal from a multitude of X-rays. The output signal is proportional to the total energy deposited by the detected X-rays.

X-ray detectors with photon counting and energy resolving capabilities are becoming common for medical X-ray applications. The photon counting detectors have an advantage since in principle the energy for each X-ray can be measured which yields additional information about the composition of the object. This information can be used to increase the image quality and/or to decrease the radiation dose.

Generally, a photon counting X-ray detector determines the energy of a photon by comparing the height of the electric pulse generated by a photon interaction in the detector material to a set of comparator voltages. These comparator voltages are also referred to as energy thresholds. Generally, the analog voltage in a comparator is set by a digital-to-analog converter (DAC). The DAC converts a digital setting sent by a controller to an analog voltage to which the heights of the photon pulses can be compared.

A photon counting detector counts the number of photons that have interacted in the detector during a measurement time. A new photon is generally identified by the fact that the height of the electric pulse exceeds the comparator voltage of at least one comparator. When a photon is identified, the event is stored by incrementing a digital counter associated with the channel.

When using several different threshold values, an energy-discriminating photon counting detector is obtained, in which the detected photons can be sorted into energy bins corresponding to the various threshold values. Sometimes, this type of photon counting detector is also referred to as a multi-bin detector. In general, the energy information allows for new kinds of images to be created, where new information is available and image artifacts inherent to conventional technology can be removed. In other words, for an energy-discriminating photon counting detector, the pulse heights are compared to a number N of programmable thresholds (T1-TN) in the comparators and are classified according to pulse-height, which in turn is proportional to energy. In other words, a photon counting detector comprising more than one comparator is here referred to as a multi-bin photon counting detector. In the case of multi-bin photon counting detector, the photon counts are stored in a set of counters, typically one for each energy threshold. For example, one count can be assigned to the highest energy threshold that the photon pulse has exceeded. In another example, counters keep track of the number of times that the photon pulse cross each energy threshold.

As an example, edge-on is a special, non-limiting design for a photon counting detector, where the X-ray sensors such as X-ray detector elements or pixels are oriented edge-on to incoming X-rays.

For example, such photon counting detectors may have pixels in at least two directions, wherein one of the directions of the edge-on photon counting detector has a component in the direction of the X-rays. Such an edge-on photon counting detector is sometimes referred to as a depth-segmented photon counting detector, having two or more depth segments of pixels in the direction of the incoming X-rays. It should be noted that one detector element may correspond to one pixel, and/or a plurality of detector elements corresponds to one pixel and/or the data signal from a plurality of detector elements may be used for one pixel.

Alternatively, the pixels may be arranged as an array (non-depth-segmented) in a direction substantially orthogonal to the direction of the incident X-rays, and each of the pixels may be oriented edge-on to the incident X-rays. In other words, the photon counting detector may be non-depth-segmented, while still arranged edge-on to the incoming X-rays.

By arranging the edge-on photon counting detector edge-on, the absorption efficiency can be increased, in which case the absorption depth can be chosen to any length, and the edge-on photon counting detector can still be fully depleted without going to very high voltages.

A conventional mechanism to detect X-ray photons through a direct semiconductor detector basically works as follows. The energy of the X-ray interactions in the detector material are converted to electron-hole pairs inside the semiconductor detector, where the number of electron-hole pairs is generally proportional to the photon energy. The electrons and holes are drifted towards the detector electrodes and backside (or vice versa). During this drift, the electrons and holes induce an electrical current in the electrode, a current which may be measured.

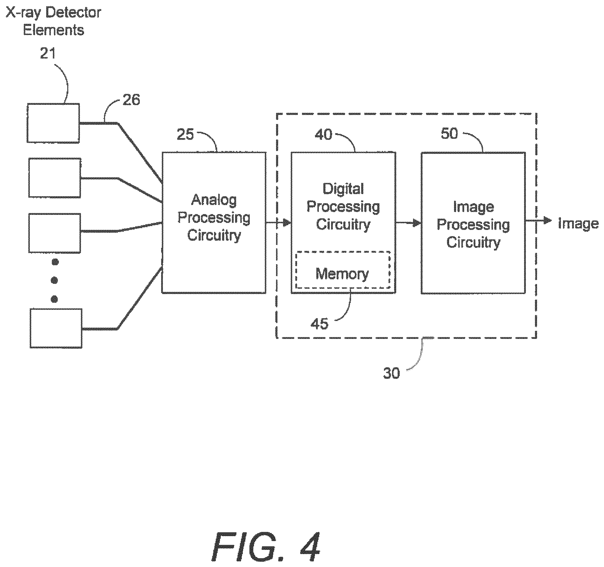

As illustrated in FIG. 4, signal(s) is/are routed via routing paths 26 from detector elements 22 of the X-ray detector to inputs of analog processing circuitry (e.g., ASICs) 25. It should be understood that the term Application Specific Integrated Circuit (ASIC) is to be interpreted broadly as any general circuit used and configured for a specific application. The ASIC processes the electric charge generated from each X-ray and converts it to digital data, which can be used to obtain measurement data such as a photon count and/or estimated energy. The ASICs are configured for connection to digital processing circuitry so the digital data may be sent to digital processing circuitry 40 and/or one or more memory circuits or components 45 and finally the data will be the input for image processing circuitry 30 or computer 50 in FIG. 2 to generate a reconstructed image.

As the number of electrons and holes from one X-ray event is proportional to the energy of the X-ray photon, the total charge in one induced current pulse is proportional to this energy. After a filtering step in the ASIC, the pulse amplitude is proportional to the total charge in the current pulse, and therefore proportional to the X-ray energy. The pulse amplitude can then be measured by comparing its value with one or more thresholds (THR) in one or more comparators (COMP), and counters are introduced by which the number of cases when a pulse is larger than the threshold value may be recorded. In this way it is possible to count and/or record the number of X-ray photons with an energy exceeding an energy corresponding to respective threshold value (THR) which has been detected within a certain time frame.

The ASIC typically samples the analog photon pulse once every Clock Cycle and registers the output of the comparators. The comparator(s) (threshold) outputs a one or a zero depending on whether the analog signal was above or below the comparator voltage. The available information at each sample is, for example, a one or a zero for each comparator representing weather the comparator has been triggered (photon pulse was higher than the threshold) or not.

In a photon counting detector, there is typically a Photon Counting Logic which determines if a new photon has been registered and, registers the photons in counter(s). In the case of a multi-bin photon counting detector, there are typically several counters, for example one for each comparator, and the photon counts are registered in the counters in accordance with an estimate of the photon energy. The logic can be implemented in several different ways. Two of the most common categories of Photon Counting Logic are the non-paralyzable counting modes, and the paralyzable counting modes. Other photon counting logics include, for example, local maxima detection, which counts, and possibly also registers the pulse height of, detected local maxima in the voltage pulse.

There are many benefits of photon counting detectors including, but not limited to high spatial resolution; less sensitivity to electronic noise; good energy resolution; and material separation capability (spectral imaging ability). However, energy integrating detectors have the advantage of high count-rate tolerance. The count-rate tolerance comes from the fact/recognition that, since the total energy of the photons is measured, adding one additional photon will always increase the output signal (within reasonable limits), regardless of the amount of photons that are currently being registered by the detector. This advantage is one of the main reasons that energy integrating detectors are the standard for medical CT today.

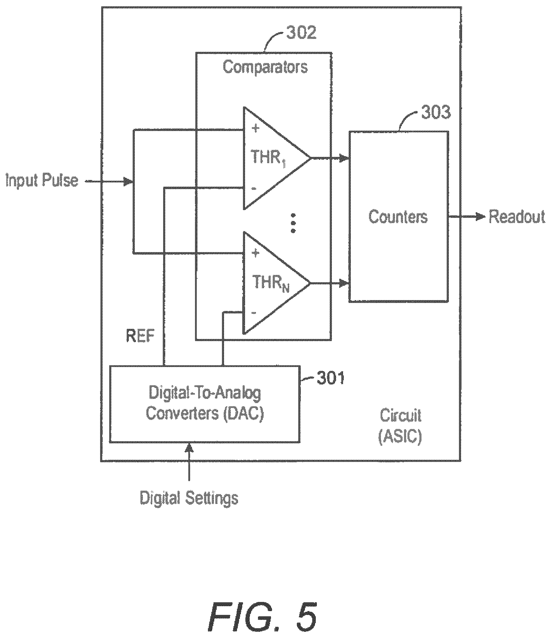

FIG. 5 shows a schematic illustration of a photon counting circuit and/or device according to an exemplary embodiment.

When a photon interacts in a semiconductor material, a cloud of electron-hole pairs is created. By applying an electric field over the detector material, the charge carriers are collected by electrodes attached to the detector material. The signal is routed from the detector elements to inputs of parallel processing circuits, e.g., ASICs. In one example, the ASIC can process the electric charge such that a voltage pulse is produced with maximum height proportional to the amount of energy deposited by the photon in the detector material.

The ASIC may include a set of comparators 302 where each comparator 302 compares the magnitude of the voltage pulse to a reference voltage. The comparator output is typically zero or one (0/1) depending on which of the two compared voltages that is larger. Here we will assume that the comparator output is one (1) if the voltage pulse is higher than the reference voltage, and zero (0) if the reference voltage is higher than the voltage pulse. Digital-to-analog converters (DACs), 301 can be used to convert digital settings, which may be supplied by the user or a control program, to reference voltages that can be used by the comparators 302. If the height of the voltage pulse exceeds the reference voltage of a specific comparator, we will refer to the comparator as triggered. Each comparator is generally associated with a digital counter 303, which is incremented based on the comparator output in accordance with the photon counting logic.

As previously mentioned, when the resulting estimated basis coefficient line integral $\hat{A}_i$ for each projection line is arranged into an image matrix, the result is a material specific projection image, also called a basis image, for each basis i. This basis image can either be viewed directly (e.g., in projection X-ray imaging) or taken as input to a reconstruction algorithm to form maps of basis coefficients $a_i$ inside the object (e.g., in CT). Anyway, the result of a basis decomposition can be regarded as one or more basis image representations, such as the basis coefficient line integrals or the basis coefficients themselves.

It will be appreciated that the mechanisms and arrangements described herein can be implemented, combined and re-arranged in a variety of ways.

For example, embodiments may be implemented in hardware, or at least partly in software for execution by suitable image processing circuitry, or a combination thereof.

The steps, functions, procedures, and/or blocks described herein may be implemented in hardware using any conventional technology, such as discrete circuit or integrated circuit technology, including both general-purpose electronic circuitry and application-specific circuitry.

Alternatively, or as a complement, at least some of the steps, functions, procedures, and/or blocks described herein may be implemented in software such as a computer program for execution by suitable image processing circuitry such as one or more processors or processing units.

In the following, non-limiting examples of specific detector module implementations will be discussed. More particularly, these examples refer to edge-on oriented detector modules and depth-segmented detector modules. Other types of detectors and detector modules may also be feasible.

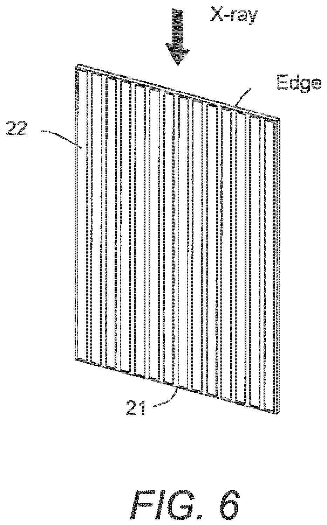

FIG. 6 is a schematic diagram illustrating an example of a semiconductor detector sub-module according to an exemplary embodiment. This is an example of a detector module 21 with a semiconductor sensor having a plurality of detector elements or pixels 22, where each detector element (or pixel) is normally based on a diode having a charge collecting electrode as a key component. The X-rays enter through the edge of the detector module.

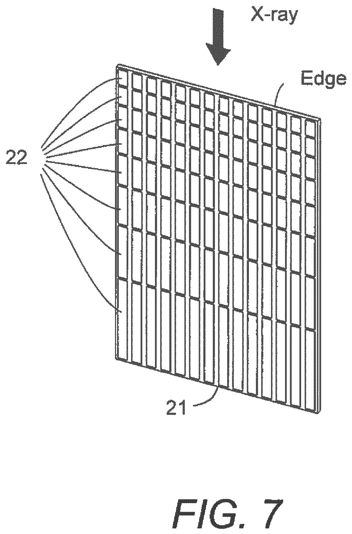

FIG. 7 is a schematic diagram illustrating an example of semiconductor detector sub-module according to another exemplary embodiment. In this example, the detector module 21 with the semiconductor sensor is also split into a plurality of depth segments or detector elements 22 in the depth direction, again assuming the X-rays enter through the edge of the detector module.

Normally, a detector element is an individual X-ray sensitive sub-element of the detector. In general, the photon interaction takes place in a detector element and the thus generated charge is collected by the corresponding electrode of the detector element.

Each detector element typically measures the incident X-ray flux as a sequence of frames. A frame is the measured data during a specified time interval, called frame time.

Depending on the detector topology, a detector element may correspond to a pixel, especially when the detector is a flat-panel detector. A depth-segmented detector may be regarded as having a number of detector strips, each strip having a number of depth segments. For such a depth-segmented detector, each depth segment may be regarded as an individual detector element, especially if each of the depth segments is associated with its own individual charge collecting electrode.

The detector strips of a depth-segmented detector normally correspond to the pixels of an ordinary flat-panel detector, and therefore sometimes also referred to as pixel strips. However, it is also possible to regard a depth-segmented detector as a three-dimensional pixel array, where each pixel corresponds to an individual depth segment/detector element.

The semiconductor sensors may be implemented as so called Multi-Chip Modules (MCMs) in the sense that the semiconductor sensors are used as base substrates for electric routing and for a number of ASICs which are attached preferably through so called flip-chip technique. The routing will include a connection for the signal from each pixel or detector element to the ASIC input as well as connections from the ASIC to external memory and/or digital data processing. Power to the ASICs may be provided through similar routing taking into account the increase in cross-section which is required for the large currents in these connections, but the power may also be provided through a separate connection. The ASICS may be positioned on the side of the active sensor and this means it can be protected from the incident X-rays if an absorbing cover is placed on top and it can also be protected from scattered X-rays from the side by positioning an absorber also in this direction.

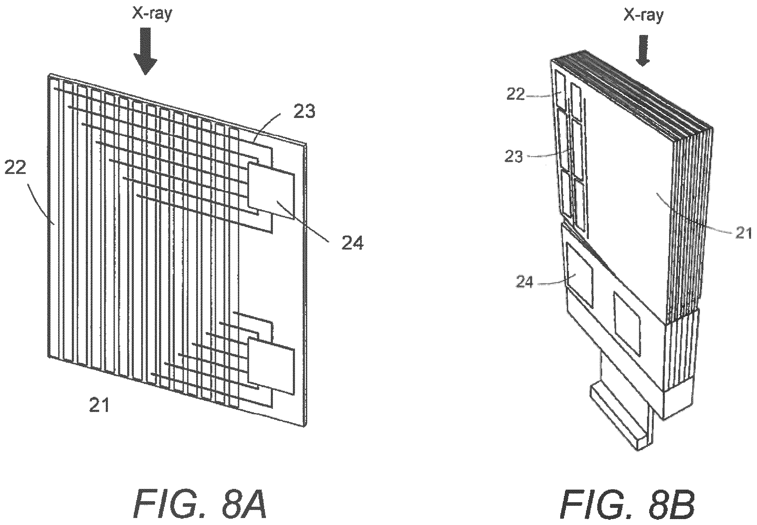

FIG. 8A is a schematic diagram illustrating a detector module implemented as a MCM similar to embodiments in U.S. Patent No. 8,183,535. In this example, it is illustrated how the semiconductor sensor 21 also can have the function of a substrate in a MCM. The signals are routed by routing paths 23 from the detector elements 22 to inputs of parallel processing circuits 24 (e.g., ASICs) that are positioned next to the active sensor area. The ASICs process the electric charge generated from each X-ray and converts it to digital data which can be used to detect a photon and/or estimate the energy of the photon. The ASICs may have their own digital processing circuitry and memory for small tasks. And, the ASICs may be configured for connection to digital processing circuitry and/or memory circuits or components located outside of the MCM and finally the data will be used as input for reconstructing an image.

However, the employment of depth segments also brings two noticeable challenges to a silicon-based photon counting detector. First, a large number of ASIC channels has to be employed to process data fed from the associated detector segments. In addition to the increased number of channels due to both the smaller pixel size and the depth segmentation, multi-energy bin further increases the data size. Second, since the given X-ray input counts are divided into smaller pixels, segments and energy bins, each bin has much lower signal and so the detector calibration/correction requires more than several orders of magnitude more calibration data to minimize statistical uncertainty.

Naturally, the several orders of magnitude larger data size slow down both data handling and pre-processing in addition to the need of larger computing resources, hard drive, memory, and central processing unit (CPU) or graphics processing unit (GPU). When the size of data is 10 Gigabytes instead of 10 Megabyte, for example, the data handling time, read and write, can take 1000 times longer.

A problem in any counting X-ray photon detector is the pile-up problem. When the flux rate of X-ray photons is high there may be problems in distinguishing between two subsequent charge pulses. As mentioned above, the pulse length after the filter depends on the shaping time. If this pulse length is larger than the time between two X-ray photon induced charge pulses, the pulses will grow together, and the two photons are not distinguishable and may be counted as one pulse. This is called pile-up. One way to avoid pile-up at high photon flux is thus to use a small shaping time, or to use depth segmentation.

For pileup calibration vector generation, the pileup calibration data needs to be pre-processed for spit correction. For material decomposition vector generation, the material decomposition data should preferably be pre-processed for both spit and pileup correction. For patient scan data, the data needs to be pre-processed for spit, pileup and material decomposition before the image reconstruction ensues. These are simplified examples to explain pre-processing since the actual pre-processing steps can include several other calibration steps as needed, like reference normalization and air calibration. The term processing may indicate only the final step in each calibration vector generation or patient scan, but it is used interchangeably in some cases.

FIG. 8B is a schematic diagram illustrating an example of a set of tiled detector sub-modules, where each detector sub-module is a depth-segmented detector sub-module and the ASICs or corresponding circuitry 24 are arranged below the detector elements 22 as seen from the direction of the incoming X-rays, allowing for routing paths 23 from the detector elements 22 to the parallel processing circuits 24 (e.g., ASICs) in the space between detector elements.

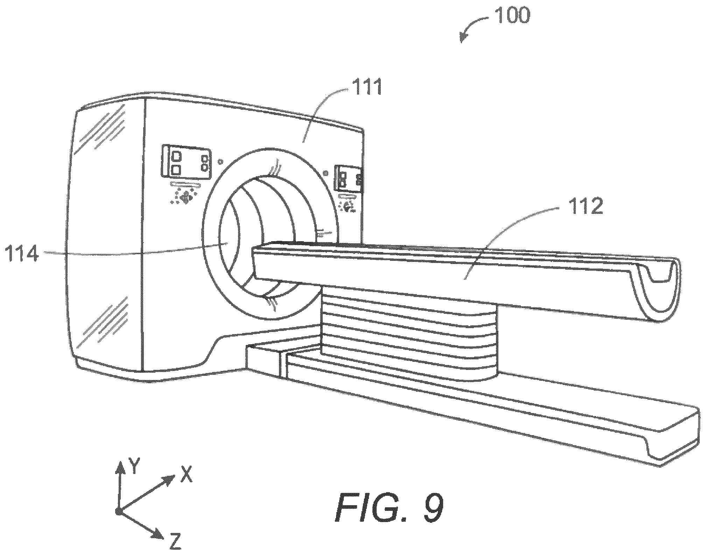

FIG. 9 is a schematic diagram illustrating an overview example of a CT imaging system. In this schematic example, the overall CT imaging system 100 comprises a gantry 111, a patient table 112 that can be inserted into an opening 114 of the gantry 111 during a patient scan and/or a calibration scan. The direction of the rotational axis of a rotating member of the gantry around a subject or patient being imaged is denoted as the z-direction. The angular direction of the CT imaging system is denoted as the x-direction, and the direction of the incident X-rays is referred to as the y-direction.

It should though be understood that the rotating member and the stationary member of the gantry do not have to be part of a CT imaging system, but may be arranged and/or configured in other ways, e.g., for linear and/or translative relative movement without rotation. As an example, the X-ray source and detector combination may be moved relative to a stationary member of the overall gantry in a linear and/or translative manner. For example, the X-ray source and detector may be moved together as an aggregate assembly unit along the table axis, commonly referred to as the z-axis. Alternatively, the patient table is moved, while the X-ray source and detector combination stands still; the relative movement is the key. This also includes geometric system configurations where the patient may be standing, e.g., in a so-called phone booth type scanner.

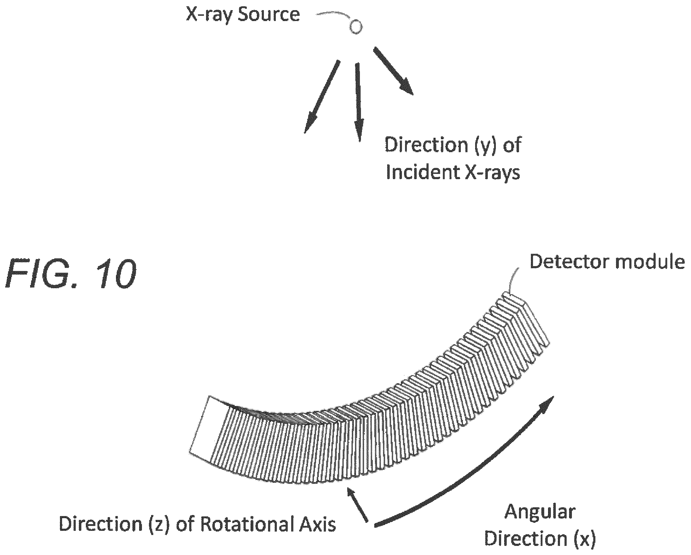

FIG. 10 is a schematic diagram illustrating an example of an overall design of an X-ray source-detector system. In this example there is shown a schematic view of an X-ray detector comprising a plurality of detector modules and an X-ray source emitting X-rays. Each detector module may have a set of detector elements defining corresponding pixels. For example, the detector modules may be edge-on detector modules, arranged side-by-side and oriented edge-on pointing back to the X-ray source, and they may be arranged in a slightly curved overall configuration. As mentioned above, the direction of the incident X-rays is referred to as the y-direction. A plurality of detector pixels in the direction of the rotational axis of the gantry (referred as z-direction) enables multi-slice image acquisition. A plurality of detector pixels in the angular direction (referred as x-direction) enables measurement of multiple projections in the same plane simultaneously and this is applied in fan/cone-beam CT. The x-direction is sometimes also referred to as the channel direction. Most detectors have detector pixels in both the slice z-direction and the angular x-direction.

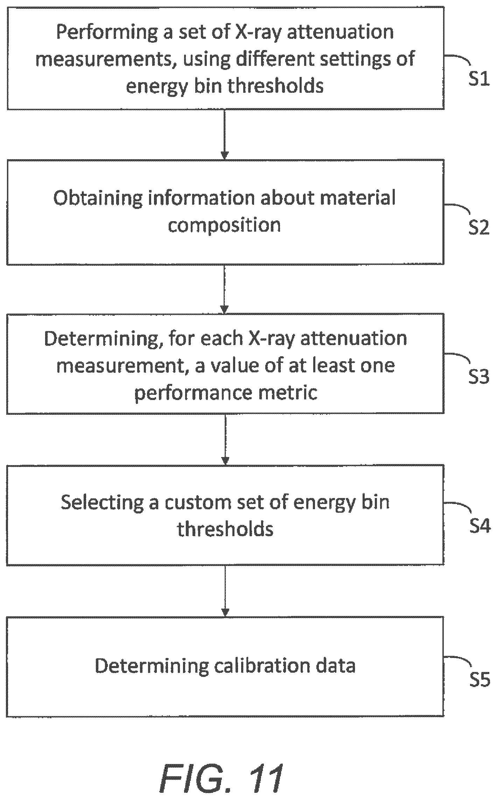

FIG. 11 is a schematic flow diagram illustrating a method for supporting calibration of an X-ray imaging system. According to an embodiment there is provided a method for supporting calibration of an X-ray imaging system, wherein the X-ray imaging system comprises a multi-bin photon counting X-ray detector having multiple energy bin thresholds. The method comprises performing S1 a set of X-ray attenuation measurements or measurement scans of at least one object, using different settings of the energy bin thresholds and obtaining S2 information about material composition related to the at least one object. The method further comprises determining S3, for each X-ray attenuation measurement or measurement scan, a value of at least one performance metric related to the X-ray imaging system and selecting S4 a custom set of energy bin thresholds based on the determined values of the at least one performance metric over the set of X-ray attenuation measurements or measurement scans. The method further comprises determining S5 calibration data coupling the selected custom set of energy bin thresholds to at least the information about material composition.

In clinical applications, thresholds are typically set before exposure, and it is thus important to select the energy threshold to achieve optimal image quality. Hence, predetermined energy thresholds are typically used with the approach of one size fit for different material, anatomies, patient sizes, phantom sizes, etc. On the contrary, the present aspect instead relates to adaptive, dynamic, or the like energy bin thresholds. By determining S5 calibration data comprising the selected custom set of energy bin thresholds, the method supports calibration of an X-ray imaging system. As a result of supporting calibration of the X-ray imaging system, the system may facilitate for increased image quality and improved patient dose efficiency. Furthermore, the method provides a more versatile and adaptable X-ray imaging system in regard to needs, requirements, and/or desires.

As mentioned above, the method comprises performing S1 a set of X-ray attenuation measurements or measurement scans. Hence, the step may comprise only performing a set of X-ray attenuation measurements, only X-ray attenuation measurement scans, or a combination of them both. These X-ray attenuation measurements and/or measurement scans may entail a high level of radiation exposure, a low level of radiation exposure, or a combination of them both. Furthermore, the measurement and/or measurement scans is to be performed of at least one object. The at least one object may for example be a phantom. Also, the step of performing S1 the set of X-ray attenuation measurements or measurement scan is performed using different settings of the energy bin thresholds. For example, a first measurement/measurement scan is performed using a first set of energy bin thresholds, and a second measurement/measurement scan is performed using a second set of energy bin thresholds, wherein the first set of energy bin thresholds is different from the second set of energy bin thresholds. By "different", it is here meant e.g., different energy ranges, different number of bins, different threshold values, and/or the like.

The method further comprises obtaining S2 information about material composition related to the at least one object. The information about material composition may be obtained by e.g., performing material decomposition of at least a portion of the object(s), receiving already determined material composition data of at least a portion of the object(s), or a combination of them both.

Furthermore, the method comprises determining S3, for each X-ray attenuation measurement or measurement scan, a value of at least one performance metric related to the X-ray imaging system. The performance metric may for example be signal-difference-to-noise ratio (SDNR) and/or signal-to-noise ratio (SNR). Hence, each X-ray attenuation measurement/measurement scan is associated with a determined value of (a) performance metric(s).

Based on the determined values of the at least one performance metric, a custom set of energy bin thresholds is selected S4 over the set of X-ray attenuation measurements or measurement scans. For example, the custom set of energy bin thresholds may correspond to the set of energy bin thresholds associated with the highest value of the performance metric(s) of all of the determined values of performance metric(s).

Additionally, the method comprises determining S5 calibration data coupling the selected custom set of energy bin thresholds to at least the information about material composition. For example, a specific material composition may correspond to a specific corresponding custom set of energy bin thresholds. The calibration data may be usable in order to calibrate an X-ray imaging system.

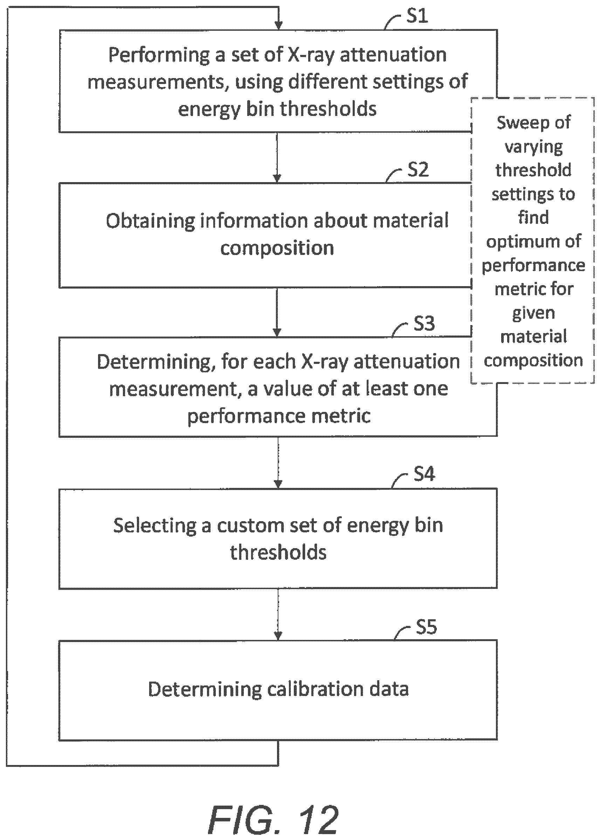

FIG. 12 is flow diagram illustrating a method for supporting calibration of an X-ray imaging system according to an exemplary embodiment. The schematic flow diagram in FIG. 12 has several features in common with the schematic flow diagram in FIG. 11, and it is hereby referred to FIG. 11 and the associated text for an increased understanding of at least some of the features and/or functions in the flow diagram.

The flow diagram in FIG. 12 illustrates an iterative process of the method for supporting calibration of an X-ray imaging system. Steps S1-S3 may correspond to a sweep of varying threshold settings in order to find an optimum threshold setting in relation to a performance metric for a given material composition of the at least one object. According to an exemplary embodiment, the method may be repeated for various material compositions. Hence, according to the example, the calibration data may comprise various information about different material compositions coupled with a corresponding selected custom set of energy bin thresholds. The method may be repeated for additional subjects, such as various settings of scan parameters, various object sizes, and/or various pixels or modules.

By way of example, the at least one object may be a phantom and the information about material composition may be obtained from known material composition data related to at least part of the phantom. Additionally, information about shape and size of the phantom may be used as complementary information together with information about the material composition. The known material composition of at least part of the object may represent a specific X-ray imaging purpose, intent, or the like. For example, the phantom may represent the material composition of a head, a chest, a pelvis, or the like, of a patient.

According to an exemplary embodiment, the information about material composition may be obtained from a material basis decomposition procedure performed based on spectral X-ray imaging data acquired during at least one of the X-ray attenuation measurements or measurement scans. Hence, (a) X-ray attenuation measurement(s) or measurement scan(s) may comprise spectral X-ray imaging data.

In a particular example, the selected custom set of energy bin thresholds may correspond to a setting of energy bin thresholds that provide an optimum of the at least one performance metric. For example, the selected custom set of energy bin thresholds may correspond to maximizing the at least one performance metric. According to another example, the selected custom set of energy bin thresholds may correspond to minimizing the at least one performance metric.

In a non-limiting example, the at least one performance metric may include at least one of an image quality metric, a metric related to the performance of detecting an image feature and a metric related to the capability of making a quantitative measurement of the composition related to the at least one object. For example, an image quality metric may relate to spatial resolution, noise, contrast, artifact level, or the like. An example of a metric related to the performance of detecting an image feature may be signal-difference-to-noise ratio (SDNR). By including a metric related to the performance of detecting an image feature, undesirable phenomena such as pile-up and charge-sharing may be reduced. Also, such metric may allow for optimal weighting of detector elements and/or detector pixels. An example of a metric related to the capability of making a quantitative measurement of the composition related to the at least one object is signal-to-noise ratio (SNR). By including a metric related to the capability of making a quantitative measurement of the object(s)'s composition, the accuracy of the obtained information about material composition related to the at least one object can be evaluated. Hence, the ability to visualize and characterize different materials may be improved. Including any of the aforementioned performance metrics may entail for more informative and valuable X-ray images obtained from the X-ray imaging system.

In another non-limiting example, the information about material composition may include at least one of material type data and material thickness data related to at least two different types of materials. Hence, the information about material composition may include data related to which at least two different types of materials are present in at least a portion of the object(s). According to a non-limiting example, the information about material composition may comprise material type data indicating that aluminium (Al) and Polyethylene (PE) are present in at least a portion of the object(s). The information about material composition may further include data related to material thickness related to at least two different types of material present in at least a portion of the object(s). For example, the information about material composition may comprise material thickness data indicating a first material thickness of a first material, and a second material thickness of a different second material. The first material thickness and the second material thickness may be the same or different.

According to an exemplary embodiment, the information about material composition may include at least one of path length information and material basis information related to at least two different types of materials. Hence, the information about material composition may include information about a first path length related to a first material, and a second path length related to a second material. The first path length and the second path length may be the same or different. Furthermore, the information about material composition may include information about material basis. For example, the material basis information may comprise density information, attenuation properties, etc, related to the at least two different types of materials. Hence, the material basis information may include information about a first material basis related to a first material and a second material basis related to a second material of the at least one object.

By way of example, each measurement or measurement scan of the set of X-ray attenuation measurements or measurement scans may be performed based on a respective unique set of energy bin thresholds to provide a sweep of varying energy bin threshold settings. Hence, a unique set of energy bin thresholds are used for each measurement or measurement scan of at least one object. By "unique set of energy bin thresholds", it is here meant that each set of energy bin thresholds is different compared to other energy bin thresholds related to a corresponding measurement or measurement scan. By "different", it is here meant e.g., different energy ranges, different number of bins, different threshold values, and/or the like. Hence, by performing each measurement or measurement scan of at least one object based on a respective unique set of energy bin thresholds, a sweep of varying energy bin threshold settings is provided.

Thus, the sweep enables selecting a custom set of energy bin thresholds corresponding to a setting of energy bin thresholds that provide an optimum of at least one performance metric for given material composition.

In a particular example, the method for supporting calibration of an X-ray imaging system may be performed to provide a set of calibration data that are usable for customizing energy bin thresholds for a multitude of different patient scans by accessing individual custom sets of energy bin thresholds for each patient scan. Hence, the set of calibration data may be used to calibrate an X-ray imaging system. A patient scan can thus be performed based on an individual custom set of energy bin thresholds. For example, the patient scan may involve a head scan, a chest scan, a pelvic scan, etc. The patient scan may correspond to a specific material composition, which is coupled to a custom set of energy bin thresholds in the determined calibration data. As the patient scan is based on individualized custom set of energy bin thresholds coupled to a corresponding material composition, the patient scan may result in an increased image quality and an efficient patient radiation dose.

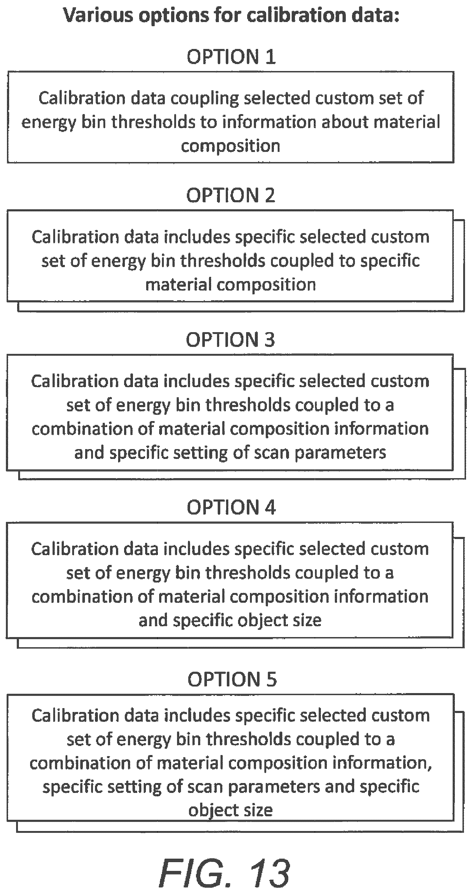

FIG. 13 is schematic diagrams illustrating various options for calibration data of an X-ray imaging system according to exemplary embodiments. The schematic diagrams in FIG. 13 have several features in common with the schematic flow diagrams in FIGS. 11 and 12, and it is hereby referred to FIGS. 11 and 12, and the associated text, for an increased understanding of at least some of the features and/or functions in the schematic diagrams.

Option 1 in FIG. 13 illustrates calibration data comprising a selected custom set of energy bin thresholds coupled to information about material composition of at least a portion of an object. Options 2 to 5 illustrates calibration data comprising a multitude of selected custom set of energy bin thresholds coupled to at least a corresponding specific material composition of at least a portion of an object. Hence, option 2 to 5 may include calibration data comprising selected custom set of energy bin thresholds of different portions of a same object and/or selected custom set of energy bin thresholds of different objects.

By way of example, the method for supporting calibration of an X-ray imaging system may be performed or repeated for various material compositions, and calibration data may be determined for each specific material composition. The calibration data for each specific material composition may include a specific selected custom set of energy bin thresholds coupled to at least material composition information related to the specific material composition. Hence, the calibration data may comprise information of several different set of material compositions, each set of material compositions corresponding to a selected custom set of energy bin thresholds.

In a particular example, the method for supporting calibration of an X-ray imaging system may further be performed or repeated for various settings of scan parameters, and calibration data may be determined for each specific setting of scan parameters. The calibration data for each specific setting of scan parameters may include a specific selected custom set of energy bin thresholds coupled to a combination of at least material composition information and the specific setting of scan parameters. Hence, the calibration data may comprise information of several different set of material compositions, each set of material compositions corresponding to a selected custom set of energy bin thresholds and a setting of scan parameters. The various settings of scan parameters may for example comprise different settings of peak voltage, electrical current flow, and/or rotational speed related to an X-ray imaging system. Taking into account the settings of scan parameters when selecting a custom set of energy bin thresholds coupled to information about material composition may further assist in optimizing patient image quality and patient radiation dose, and the trade-off therebetween. Also, optimal scan parameters with respect to a custom set of energy bin thresholds coupled to at least information about material composition may be different for different X-ray imaging systems. Hence, the method provides a more versatile and adaptable X-ray imaging system in regard to needs, requirements, and/or techniques.

In another example, the method for supporting calibration of an X-ray imaging system may further be performed or repeated for various object sizes of the at least one object, and calibration data may be determined for each specific object size. The calibration data for each specific object size may include a specific selected custom set of energy bin thresholds coupled to a combination of at least material composition information and object size. Hence, the calibration data may comprise information of several different set of material compositions, each set of material compositions corresponding a selected custom set of energy bin thresholds and a specific object size. The object size may comprise at least one of length, width, and thickness information of each object of the at least one object. Taking into account the size of the object when selecting a custom set of energy bin thresholds coupled to information about material composition may further assist in optimizing patient image quality and patient radiation dose. For example, a head scan of a child may benefit, in terms of e.g., patient image quality and patient radiation dose, by the use of a custom set of energy bin thresholds different from a custom set of energy bin threshold used to perform a head scan of an adult.

In a non-limiting example, the method for supporting calibration of an X-ray imaging system may be performed for a set of detector pixels or detector modules of the multi-bin photon counting X-ray detector. The calibration data including a specific selected custom set of energy bin thresholds may be determined for each of the detector pixels or detector modules. Thus, the potential varying characteristics and properties of the set of detector pixels and/or the detector modules may be taken into account when selecting a custom set of energy bin thresholds coupled to information about material composition. Hence, the energy bin thresholds may be customized on a pixel-by-pixel basis and/or on a module-by-module basis. This is advantageous in terms of optimizing e.g., patient image quality and patient radiation dose as each detector pixel may have different detection efficiency and performance. Customizing the energy bin thresholds based on a module-by-module basis may also be advantageous as the module geometric efficiency, module position and/or module size may be considered. Hence, the method provides a more versatile and adaptable X-ray imaging system in regard to needs, requirements, and/or techniques.

According to an exemplary embodiment, the method for supporting calibration of an X-ray imaging system may further comprise the step of storing S6 the calibration data in a look-up data structure or function. This look-up data structure or function may for example be usable by the X-ray imaging system itself, be saved for later use, and/or be transferred to another entity such as another X-ray imaging system.

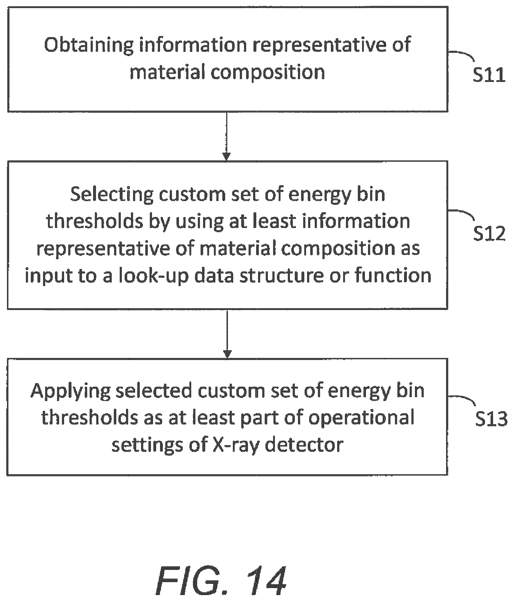

FIG. 14 is a schematic flow diagram illustrating a method for adjusting operational settings for an X-ray imaging system. According to an aspect, there is provided a method for adjusting operational settings for an X-ray imaging system comprising a multi-bin photon counting X-ray detector having multiple energy bin thresholds. The method comprises obtaining S11 information representative of material composition related to at least one object and selecting S12 a custom set of energy bin thresholds based on at least the information representative of material composition by using at least the information representative of material composition as input to a look-up data structure or function holding calibration data for retrieval of a set of energy bin thresholds that matches or corresponds to the information representative of material composition. The method further comprises applying S13 the selected custom set of energy bin thresholds as at least part of operational settings of the multi-bin photon counting X-ray detector of the X-ray imaging system.

Hence, the method comprises obtaining S11 material composition information related to at least one object. The at least one object may for example be a phantom or a patient. More specifically, the at least one object may be a head, chest, pelvic, etc. of the patient. Based on the material composition information, a custom set of energy bin thresholds may be selected S12, by using calibration data coupling the material composition information to the custom set of energy bin thresholds. The selected custom set of energy bin thresholds are applied S13 to operational settings of the multi-bin photon counting X-ray detector of the X-ray imaging system.

By way of example, the calibration data in the look-up data structure or function may be determined according to the aforementioned method for supporting calibration of an X-ray imaging system.

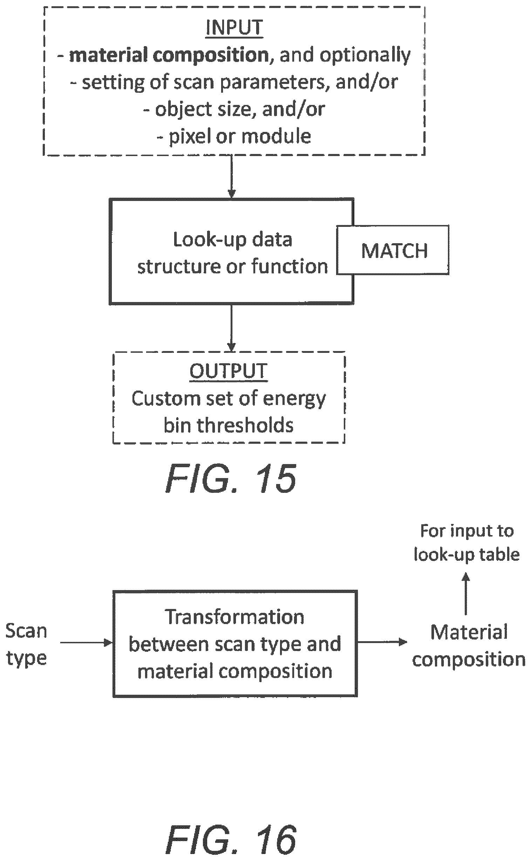

FIG. 15 is a schematic diagram illustrating an example of the look-up data function or structure. As illustrated in FIG. 15, the look-up data structure or function compares input data to calibration data of the look-up data structure of function. The input data comprises at least information about material composition of the at least one object. Furthermore, a matching or corresponding custom set of energy bin thresholds is retrieved as output from the look-up data structure or function. The outputted custom set of energy bin thresholds may be applied S13 to operational settings of of the multi-bin photon counting X-ray detector of the X-ray imaging system.

According to an exemplary embodiment, selecting a custom set of energy bin thresholds may further be based on a setting of scan parameters by using at least the setting of scan parameters as input to the look-up data structure or function holding calibration data for retrieval of a set of energy bin thresholds that matches or corresponds to a combination of at least the information representative of material composition and the setting of scan parameters. Hence, both information about material composition and a setting of scan parameters may be given as input to the look-up data structure or function.

In a particular example, selecting a custom set of energy bin thresholds may further be based on an object size of the at least one object by using at least the object size as input to the look-up data structure or function holding calibration data for retrieval of a set of energy bin thresholds that matches or corresponds to a combination of at least the information representative of material composition and the object size. Hence, both information about material composition and of an object size of the object(s) may be given as input to the look-up data structure or function. For example, the dimensions, size, or the like of an abdomen of a patient may be used as input to the look-up data structure or function.

In a non-limiting example, selecting a custom set of energy bin thresholds may further be based on location information related to a pixel or detector module in the X-ray detector by using the location information as input to the look-up data structure or function holding calibration data for retrieval of a set of energy bin thresholds that matches or corresponds to a combination of at least the information representative of material composition and the location information. The location information related to a pixel or detector module in the X-ray detector may indicate the position of the pixel or detector module in the X-ray detector. This may be advantageous in terms of optimizing e.g., patient image quality and patient radiation dose. For example, each detector pixel may have different detection efficiency and performance. Furthermore, detector modules may have different geometric efficiency based on detector module position and/or detector module size. As described, both information about material composition and of location information related to a pixel and/or a detector module may be given as input to the look-up data structure or function.

It should be noted that the input data to the look-up data structure or function comprises information about material composition. The input data may further comprise any combination of a setting of scan parameter, object size, and location information related to a pixel and/or a detector module.

FIG. 16 is a schematic diagram illustrating an example of how to obtain info about material composition based on a scan type. By way of example, the method for adjusting operational settings for an X-ray imaging system may be performed for a given scan, according to at least one of prior to the scan and during the scan. Operational settings for the X-ray imaging system may for example comprise scan parameters and energy bin thresholds. The selected custom set of energy bin thresholds retrieved from the look-up data structure or function may thus provide an initial guess for energy bin thresholds. The operational settings for the X-ray imaging system may be further adjusted during the scan, i.e., on-the-fly in real-time or near real-time. For example, the method may consider count rates, charge-sharing, pile-up and/or system noise during the scan, in order to further adjust the operational settings for the X-ray imaging system.

According to an exemplary embodiment, the information representative of material composition may be derived based on a specific type of scan to be performed by the X-ray imaging system. As illustrated in FIG. 16, a scan type may be given as input to a transformation function or structure. The transformation function or structure transforms the scan type to corresponding information representative of material composition. The corresponding information representative of material composition may be an approximation. The (approximative) information related to a material composition may be given as input to the look-up data structure, function, table, or the like.

In a non-limiting example, the specific type of scan may be selected from a set of different scan types including at least one of the following: head scan, chest scan, abdominal scan, pelvic scan, spinal scan, cardiac scan. Information representative of material composition may be derived based on the selected type of scan and used as input to the look-up data structure or function for retrieval of the custom set of energy bin thresholds. Hence, the custom set of energy bin thresholds may be customized for the material composition corresponding to a selected type of scan. The scan type may for example be chosen by an operator of the X-ray imaging system. It should be noted that additional scan types than mentioned herein may be equally feasible.

In another non-limiting example, the information representative of material composition may be derived based on material basis decomposition performed by the X-ray imaging system using spectral X-ray imaging data obtained by the multi-bin photon counting X-ray detector. According to a non-limiting example, material basis decomposition is performed on the at least one object to be scanned, for example a patient, in order to obtain information representative of material composition of the object(s).

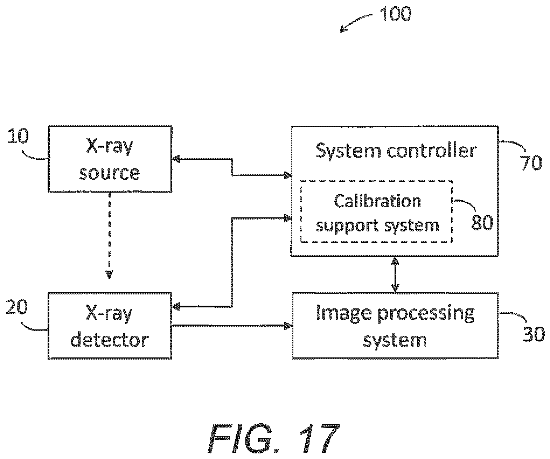

FIG. 17 is a schematic block diagram illustrating a system for supporting calibration of an X-ray imaging system. The X-ray imaging system in FIG. 17 has several features in common with the X-ray imaging system in FIGS. 1-10, and it is hereby referred to FIG. 1-10 and the associated text for an increased understanding of at least some of the features and/or functions in the X-ray imaging system. According to an aspect, there is provided a system 80 for supporting calibration of an X-ray imaging system comprising a multi-bin photon counting X-ray detector 20 having multiple energy bin thresholds, wherein the system 80 is configured to perform the aforementioned method for supporting calibration of an X-ray imaging system. The X-ray imaging system 100 in FIG. 17 may be the same X-ray imaging system 100 as previously described herein. As illustrated in FIG. 17, the calibration support system 80 may be integrated in a system controller 70 of the X-ray imaging system 100. Alternatively, the calibration support system 80 may be arranged externally and connectable relative the system controller 70.

According to an aspect, there is provided a Computed Tomography (CT) system 100 comprising the system 80 for supporting calibration of an X-ray imaging system. The CT system 100 may be the same as the CT system 100 previously described herein.

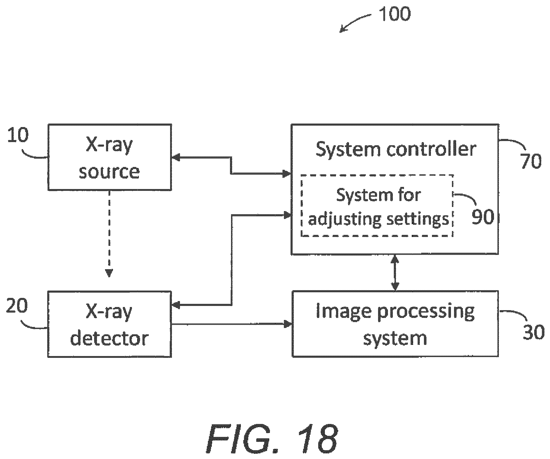

FIG. 18 is a schematic block diagram illustrating a system 90 for adjusting operational settings for an X-ray imaging system. The X-ray imaging system 100 in FIG. 18 has several features in common with the X-ray imaging system 100 in FIGS. 1-10 and 17, and it is hereby referred to FIG. 1-10 and 17 and the associated text for an increased understanding of at least some of the features and/or functions in the X-ray imaging system 100.

According to an aspect, there is provided a system 90 for adjusting operational settings for an X-ray imaging system comprising a multi-bin photon counting X-ray detector 20 having multiple energy bin thresholds, wherein the system is configured to perform the aforementioned method of for adjusting operational settings for an X-ray imaging system. The system 90 for adjusting operational settings may be integrated in a system controller 70 of the X-ray imaging system 100. Alternatively, the system 90 for adjusting operational settings for an X-ray imaging system may be arranged externally and connectable relative the system controller 70.

According to an aspect, there is provided a Computed Tomography (CT) system 100 comprising the system 90 for adjusting operational settings for an X-ray imaging system. The CT system 100 may be the same as the CT system 100 previously described herein.

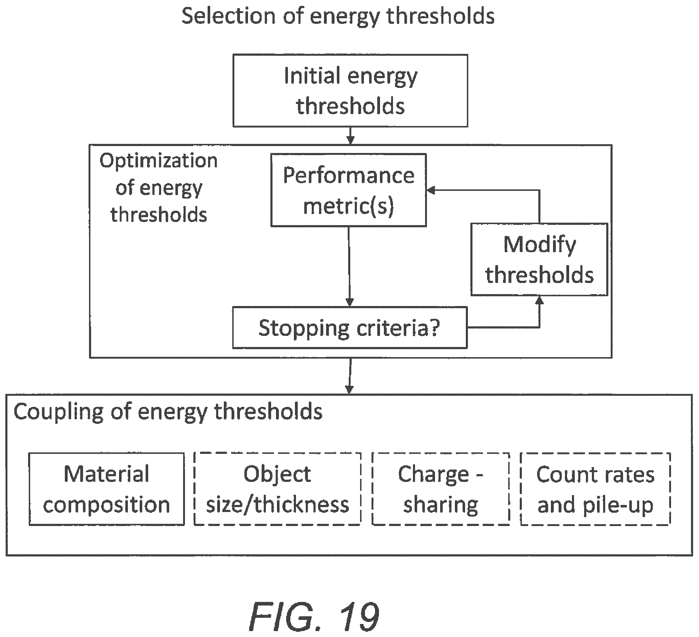

For a better understanding of the proposed technology, reference will now be made to FIGS. 19-25 relating to non-limiting examples of particular embodiments.

FIG. 19 is a schematic flow diagram illustrating a method for adjusting operational settings for an X-ray imaging system according to an exemplary embodiment. More specifically, FIG. 19 schematically illustrates the step of selecting S4 a custom set of energy bin thresholds based on determined values of at least one performance metric, according to the method for supporting calibration of an X-ray imaging system. According to the exemplifying embodiment, an initial X-ray attenuation measurement or measurement scan of at least one object is performed using an initial set of energy bin thresholds. For the X-ray attenuation measurement or measurement scan, at least one performance metric is determined.

During the optimization of energy bin thresholds, in order to select a custom set of energy bin thresholds corresponding to an (optimal) performance metric, a set of X-ray attenuation measurements or measurement scans of the at least one object using different, modified, adjusted, etc., settings of the energy bin thresholds is performed. The optimization of energy bin thresholds may be stopped if at least one stopping criterion is reached or exceeded. For example, the stopping criteria may comprise a predetermined threshold for the performance metric and/or a maximal number of iterations related to the optimization. Calibration data is thereafter determined, coupling the selected custom set of energy bin thresholds to at least obtained information about material composition. Optionally, the calibration data may comprise coupling between the custom set of energy bin thresholds, information about material composition, and the size/thickness of the object. Additionally, further parameters, aspects, settings, or the like may be considered when determining the calibration data. For example, charge-sharing, count rates, and/or pile-up may also be considered when evaluating the performance metric in order to select a custom set of energy bin thresholds.

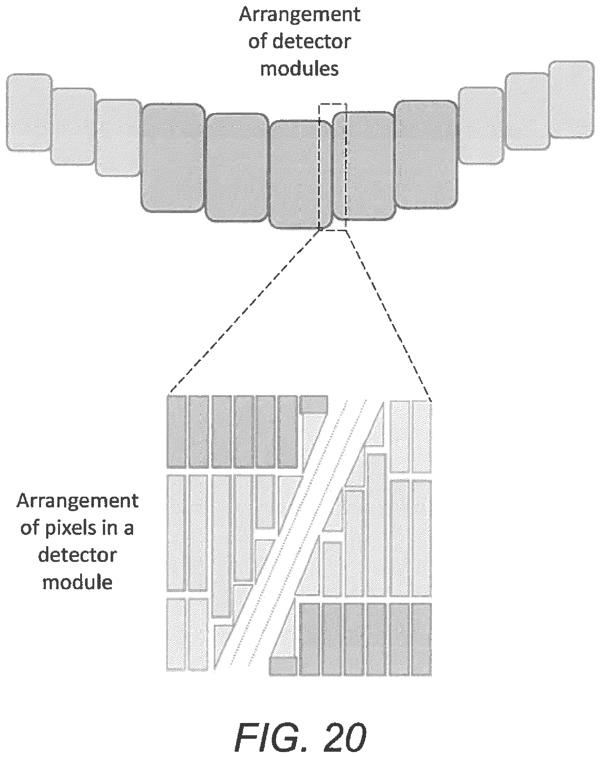

FIG. 20 is a schematic diagram illustrating an example of an arrangement of detector sub-modules and an associated arrangement of detector sub-pixels according to an exemplary embodiment. Determining calibration data according to the method for supporting calibration of an X-ray imaging system is based on performing S1 a set of X-ray attenuation measurements or measurement scans of at least one object, using different settings of the energy bin thresholds. Acquiring the set of X-ray attenuation measurements or measurement scans may further be performed for a set of detector pixels or detector modules of the multi-bin photon counting X-ray detector of the X-ray imaging system. Hence, the calibration data including a specific selected custom set of energy bin thresholds may be determined for each of the detector pixels and/or detector modules. In other words, the custom set of energy bin thresholds may be adaptable on a pixel-by-pixel basis and/or on a module-by-module basis. For example, the custom set of energy bin thresholds may be selected based on module geometric efficiency, module position, and/or module size. Also, the custom set of energy bin thresholds may be selected based on pixel efficiency. This may be advantageous as each detector pixel may have different detection efficiency and/or performance.

Furthermore, selecting S12 a custom set of energy bin thresholds may be further based on location information related to a detector pixel and/or a detector module in the X-ray detector. The location information may be used as input to the look-up data structure or function holding, comprising, or the like the calibration data for retrieval of a set of energy bin thresholds that matches or corresponds to a combination of at least the material composition information and the location information.

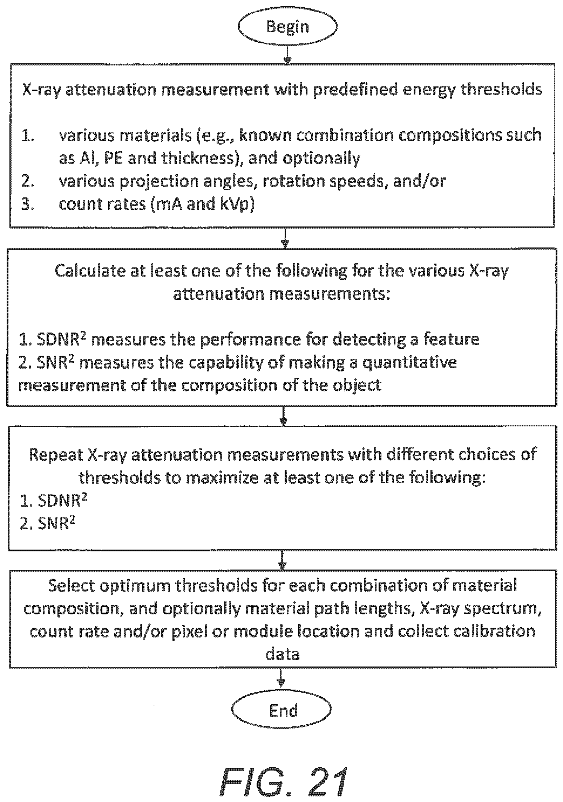

FIG. 21 is a schematic flow diagram illustrating a method for supporting calibration of an X-ray imaging system according to an exemplary embodiment. As illustrated in FIG. 21, X-ray attenuation measurements or measurement scans are performed of at least one object using a predefined, predetermined, or the like setting of a set of energy bin thresholds. The material composition of the at least one object may be known. Optionally, the X-ray attenuation measurements or measurement scans may be performed for various materials, projection angles, rotation speeds, and/or count rates. For each measurement or measurement scan, a value of at least one performance metric related to the X-ray imaging may be determined. As shown in FIG. 21, the at least one performance metric may comprise $SDNR^2$ and/or $SNR^2$. The X-ray attenuation measurements or measurement scans may be repeated using different settings of the energy bin threshold in order to maximize the at least one performance metric. An optimum, customized, or the like set of energy bin thresholds may be selected for each material composition. The custom set of energy bin thresholds coupled to material composition may be stored as calibration data. Selecting the custom set of energy bin thresholds may further be based on at least material path lengths, X-ray spectrum, count rate, detector pixel location, and/or detector module location.

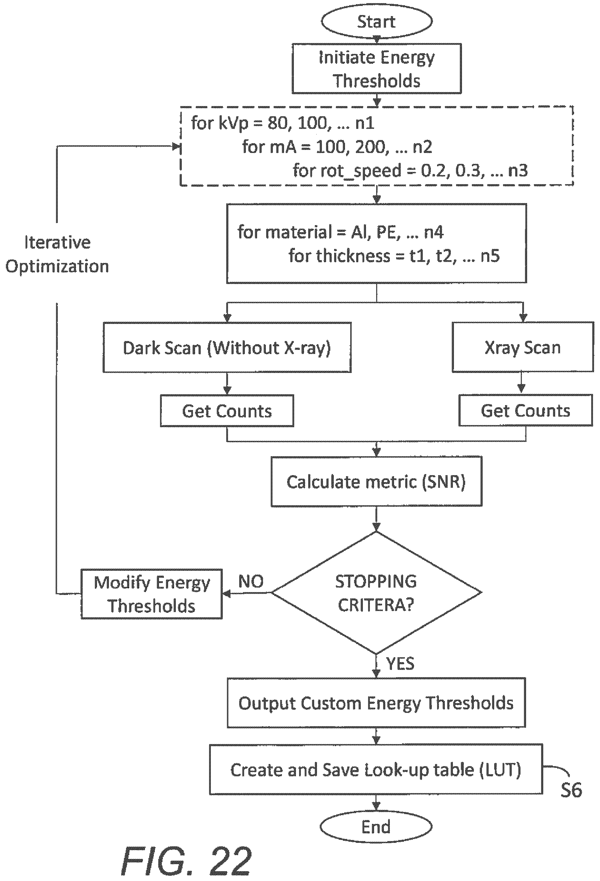

FIG. 22 is a schematic flow diagram illustrating a method for supporting calibration of an X-ray imaging system according to an exemplary embodiment. As FIG. 22 illustrates, the method for supporting calibration of an X-ray imaging system comprises an iterative optimization for selecting S4 a custom set of energy bin thresholds. Furthermore, the step of performing S1 a set of X-ray attenuation measurements or measurement scans of at least one object, using different settings of the energy bin thresholds, may further comprise at least one dark scan, reference scan, noise scan, or the like. The embodiment may be advantageous because the at least one dark scan may further support calibration of the X-ray imaging system by considering baseline noise and signals inherent to the X-ray imaging system itself. Hence, taking at least one dark scan into account when determining the at least one performance metric of the X-ray attenuation measurements or measurement scans may improve supporting calibration of the X-ray imaging system. The method further comprises storing S6 calibration data comprising the custom set of energy bin threshold in a look-up data structure or function.

Furthermore, as illustrated in FIG. 22, at least one stopping criterion may be present in the method, to evaluate if the iterative optimization should continue or not. For example, the stopping criterion may comprise evaluating if the at least one performance metric reaches or exceeds a predetermined acceptable threshold value. FIG. 22 illustrates how an initial set of energy bin thresholds is used for performing an X-ray attenuation measurement or measurement scan. Hence, if the at least one stopping criterion is not fulfilled, the set of energy bin thresholds may be adjusted, modified, etc., for performing at least one additional X-ray attenuation measurement or measurement scan.

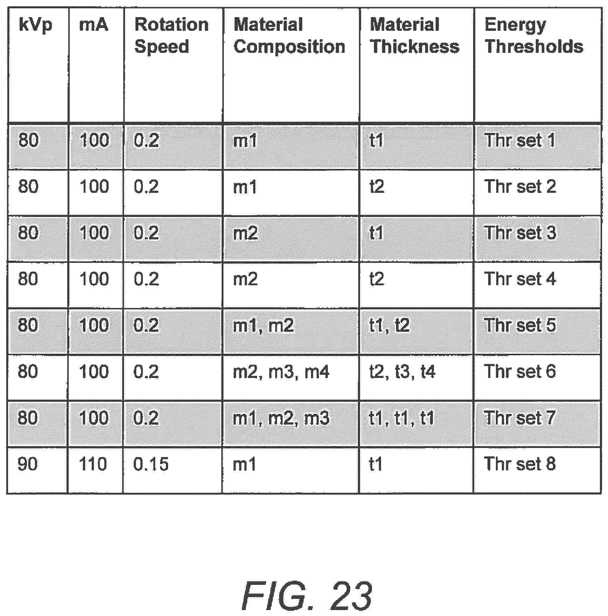

FIG. 23 is a table illustrating examples of calibration data for supporting calibration of an X-ray imaging system according to an exemplary embodiment. The table of FIG. 23 illustrates various examples of the coupling between values of operational settings corresponding to a custom set of energy bin thresholds. The operational settings may for example comprise information about material composition and/or scan parameters. The scan parameters may for example comprise peak voltage (kVp) applied to the X-ray tube, current (mA), and/or rotation speed of an X-ray imaging system. Information about material composition may comprise specific material composition of at least a portion of an object and the thickness of each the material. When selecting a custom set of energy bin thresholds, information representative of material composition may be used as input to a look-up data structure or function holding calibration data. Additional information such as scan parameters may also be used as input to the look-up data structure or function. For example, the loop-up data structure or function may comprise the table of FIG. 23. The look-up data structure or function may comprise a matching function configured to match the input to corresponding calibration data. Hence, the input comprising at least information representative of material composition may be matched to corresponding calibration data. The resulting output from the look-up data structure or function is therefore a set of energy bin thresholds that matches or corresponds to at least the information representative of material composition.

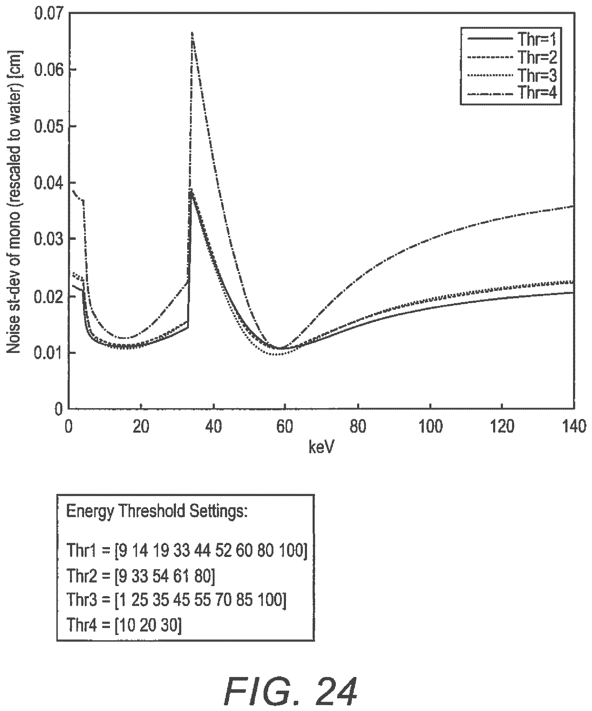

FIG. 24 is a graph illustrating a relation between keV and noise standard deviation of mono energy for different energy bin thresholds according to an exemplary embodiment. According to the exemplifying graph of FIG. 24, four different sets of energy bin thresholds have been used, each corresponding to a respective sub-graph. The basis materials of the object resulting in the graph in FIG. 24 comprises at least water and iodine as an exemplifying embodiment. Threshold sets 1-3 (Thr1, Thr2, Thr3) are all optimized according to the method of any of the claim 1 to 14, whilst threshold set 4 (Thr4) is not. According to the exemplifying graph, threshold set 1 (Thr1) resulted in the most desirable performance in regard to noise standard deviation of mono energy. Threshold set 4 (Thr4) on the other hand resulted in the poorest performance.

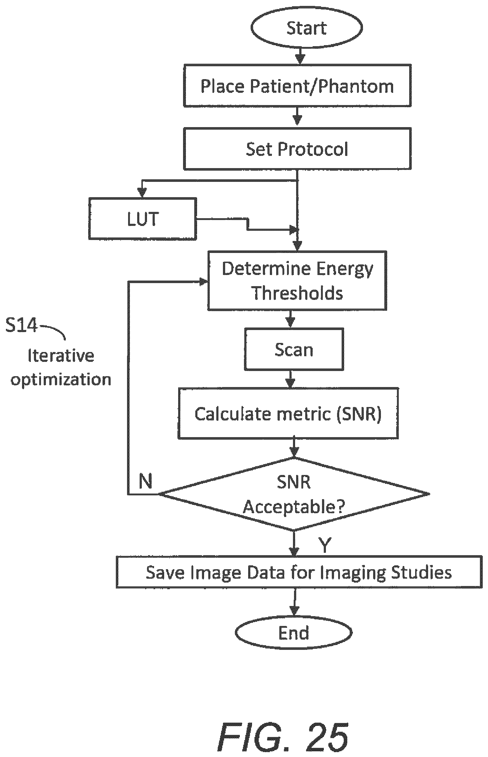

FIG. 25 is a schematic flow diagram illustrating a method for adjusting operational settings for an X-ray imaging system according to an exemplary embodiment. By way of example, the method of adjusting operational settings for an X-ray imaging system may further comprise iteratively adapting S14, during a patient scan, the custom set of energy bin thresholds towards a maximum of at least one performance metric related to the X-ray imaging system using the information representative of material composition that is derived based on material basis decomposition. In other words, after performing a scan based on the retrieved custom set of energy bin thresholds from the look-up data structure or function, represented here by "LUT", the set of energy bin thresholds may be further adjusted, adapted, optimized, or the like. The step of iteratively adapting S14 the set of energy bin thresholds may thus maximize the performance metric. Furthermore, the step S14 may further comprise iteratively adapting, adjusting, optimizing, or the like, scan parameters of the associated X-ray imaging system towards a maximum of at least one performance metric related to the X-ray imaging system. Additionally, charge-sharing, count rates, and/or pile-up may also be considered when selecting a custom set of energy bin thresholds. This is advantageous in that these phenomena may vary for different X-ray imaging systems. Also, the phenomena may vary for each scan. The further optimized custom set of energy bin thresholds may be applied as at least part of operational settings of the multi-bin photon counting X-ray detector of the X-ray imaging system.

According to an exemplary embodiment, the method for adjusting operational settings for an X-ray imaging system comprising the step of iteratively adapting the custom set of energy bin thresholds may be performed if the performance metric for the set of energy bin thresholds initially retrieved from the look-up data structure or function is below a predetermined value. The step of iteratively adapting the custom set of energy bin thresholds may be repeated until an acceptable performance metric has been obtained. The radiation dose of the scan performed in FIG. 25 may be adjusted based on for example the physical state of the patient. For example, the radiation dose may comprise a low radiation dose, a high radiation dose, or a radiation dose therebetween.

In a particular implementation example, the proposed technology may be performed on-the-fly in real-time or near real-time to adaptively select energy bin thresholds for a particular patient scan.

As mentioned, at least some of the steps, functions, procedures, and/or blocks described herein may be implemented in software such as a computer program for execution by suitable processing circuitry such as one or more processors or processing units.

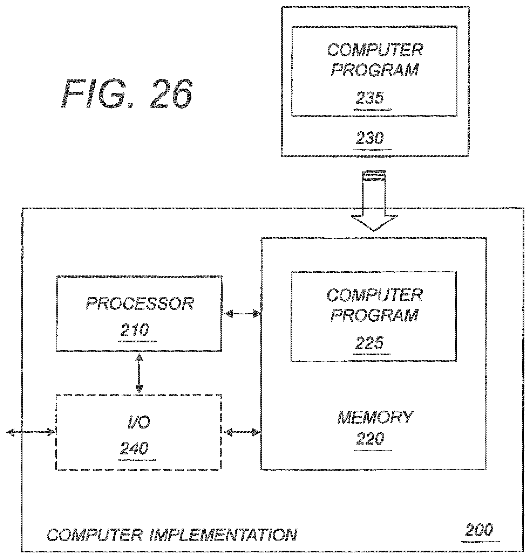

FIG. 26 is a schematic diagram illustrating an example of a computer implementation according to an embodiment. In this particular example, the system 200 comprises a processor 210 and a memory 220, the memory comprising instructions executable by the processor, whereby the processor is operative to perform the steps and/or actions described herein. The instructions are typically organized as a computer program 225; 235, which may be preconfigured in the memory 220 or downloaded from an external memory device 230. Optionally, the system 200 comprises an input/output interface 240 that may be interconnected to the processor(s) 210 and/or the memory 220 to enable input and/or output of relevant data such as input parameter(s) and/or resulting output parameter(s). In a particular example, the memory 220 comprises a set of instructions executable by the processor, whereby the processor is operative to perform the steps and/or actions described herein.

The term 'processor' should be interpreted in a general sense as any system or device capable of executing program code or computer program instructions to perform a particular processing, determining or computing task. The processing circuitry including one or more processors is thus configured to perform, when executing the computer program, well-defined processing tasks such as those described herein. The processing circuitry does not have to be dedicated to only execute the above-described steps, functions, procedure and/or blocks, but may also execute other tasks.

The proposed technology also provides a computer-program product comprising a computer-readable medium 220; 230 having stored thereon such a computer program.

By way of example, the software or computer program 225; 235 may be realized as a computer program product, which is normally carried or stored on a computer-readable medium 220; 230, in particular a non-volatile medium. The computer-readable medium may include one or more removable or non-removable memory devices including, but not limited to a Read-Only Memory (ROM), a Random Access Memory (RAM), a Compact Disc (CD), a Digital Versatile Disc (DVD), a Blu-ray disc, a Universal Serial Bus (USB) memory, a Hard Disk Drive (HDD) storage device, a flash memory, a magnetic tape, or any other conventional memory device. The computer program may thus be loaded into the operating memory of a computer or equivalent processing device for execution by the processing circuitry thereof.

Method flows may be regarded as a computer action flows, when performed by one or more processors. A corresponding device, system and/or apparatus may be defined as a group of function modules, where each step performed by the processor corresponds to a function module. In this case, the function modules are implemented as a computer program running on the processor. Hence, the device, system and/or apparatus may alternatively be defined as a group of function modules, where the function modules are implemented as a computer program running on at least one processor.

The computer program residing in memory may thus be organized as appropriate function modules configured to perform, when executed by the processor, at least part of the steps and/or tasks described herein.

Alternatively, it is possible to realize the modules predominantly by hardware modules, or alternatively by hardware. The extent of software versus hardware is purely implementation selection.

Embodiments of the present disclosure shown in the drawings and described above are example embodiments only and are not intended to limit the scope of the appended claims, including any equivalents as included within the scope of the claims. It will be understood by those skilled in the art that various modifications, combinations, and changes may be made to the embodiments without departing from the present scope as defined by the appended claims. It is intended that any combination of non-mutually exclusive features described herein are within the scope of the present disclosure. That is, features of the described embodiments can be combined with any appropriate aspect described above and optional features of any one aspect can be combined with any other appropriate aspect. Similarly, features set forth in dependent claims can be combined with non-mutually exclusive features of other dependent claims, particularly where the dependent claims depend on the same independent claim. Single claim dependencies may have been used as practice in some jurisdictions require them, but this should not be taken to mean that the features in the dependent claims are mutually exclusive.

It is further noted that the inventive concepts relate to all possible combinations of features unless explicitly stated otherwise. In particular, different part solutions in the different embodiments can be combined in other configurations, where technically possible.

**Claims**

1. A method for supporting calibration of an X-ray imaging system, wherein the X-ray imaging system comprises a multi-bin photon counting X-ray detector having multiple energy bin thresholds, the method comprising:

   performing (S1) a set of X-ray attenuation measurements or measurement scans of at least one object, using different settings of the energy bin thresholds;
   obtaining (S2) information about material composition related to the at least one object;
   determining (S3), for each X-ray attenuation measurement or measurement scan, a value of at least one performance metric related to the X-ray imaging system;
   selecting (S4) a custom set of energy bin thresholds based on the determined values of the at least one performance metric over the set of X-ray attenuation measurements or measurement scans; and
   determining (S5) calibration data coupling the selected custom set of energy bin thresholds to at least the information about material composition.

2. The method of claim 1, wherein the method is performed or repeated for various material compositions, and calibration data is determined for each specific material composition, wherein the calibration data for each specific material composition includes a specific selected custom set of energy bin thresholds coupled to at least material composition information related to the specific material composition.

17

3. The method of claims 1, wherein the method is further performed or repeated for various settings of scan parameters, and calibration data is determined for each specific setting of scan parameters, wherein the calibration data for each specific setting of scan parameters includes a specific selected custom set of energy bin thresholds coupled to a combination of at least material composition information and the specific setting of scan parameters.

4. The method of claims 1, wherein the method is further performed or repeated for various object sizes of the at least one object, and calibration data is determined for each specific object size, wherein the calibration data for each specific object size includes a specific selected custom set of energy bin thresholds coupled to a combination of at least material composition information and object size.

5. The method of any of the claims 1, wherein the method is performed for a set of detector pixels or detector modules of the multi-bin photon counting X-ray detector, and calibration data including a specific selected custom set of energy bin thresholds is determined for each of the detector pixels or detector modules.

6. The method of any of the claims 1, wherein the at least one object is a phantom and the information about material composition is obtained from known material composition data related to at least part of the phantom.

7. The method of any of the claims 1, wherein the information about material composition is obtained from a material basis decomposition procedure performed based on spectral X-ray imaging data acquired during at least one of the X-ray attenuation measurements or measurement scans.

8. The method of any of the claims 1, wherein the selected custom set of energy bin thresholds corresponds to a setting of energy bin thresholds that provide an optimum of the at least one performance metric.

9. The method of any of the claims 1, wherein the at least one performance metric includes at least one of an image quality metric, a metric related to the performance of detecting an image feature and a metric related to the capability of making a quantitative measurement of the composition related to the at least one object.

10. The method of any of the claims 1, wherein the information about material composition includes at least one of material type data and material thickness data related to at least two different types of materials.

11. The method of any of the claims 1, wherein the information about material composition includes at least one of path length information and material basis information related to at least two different types of materials.

12. The method of any of the claims 1, further comprising the step of storing (S6) the calibration data in a look-up data structure or function.

13. The method of any of the claims 1, wherein each measurement or measurement scan of the set of X-ray attenuation measurements or measurement scans is performed based on a respective unique set of energy bin thresholds to provide a sweep of varying energy bin threshold settings.

14. The method of any of the claims 1, wherein the method is performed to provide a set of calibration data that are usable for customizing energy bin thresholds for a multitude of different patient scans by accessing individual custom sets of energy bin thresholds for each patient scan.

100

X-ray
Source

X-ray
Detector

20

Object

30

10

Image Processing
System

FIG. 1A

FIG. 1B

100

Image Processing System

Computer
50

Digital Processing Circuitry
40

30

Analog Processing Circuitry
25

20

X-ray Detector

Gantry 11

Object

X-ray Source

10

FIG. 2

*FIG. 3*

X-ray Detector
Elements

21

26

25

40

50

Analog
Processing
Circuitry

Digital
Processing
Circuitry

Memory

45

Image
Processing
Circuitry

Image

30

# FIG. 4

FIG. 5

FIG. 6

FIG. 7

X-ray

21

22

23

24

*FIG. 8B*

X-ray

23

24

22

21

*FIG. 8A*

FIG. 9

EP 4 667 979 A1

EP 4 667 979 A1

X-ray Source

Direction (y) of
Incident X-rays

FIG. 10

Detector module

Direction (z) of Rotational Axis

Angular
Direction (x)

Performing a set of X-ray attenuation measurements, using different settings of energy bin thresholds — S1

Obtaining information about material composition — S2

Determining, for each X-ray attenuation measurement, a value of at least one performance metric — S3

Selecting a custom set of energy bin thresholds — S4

Determining calibration data — S5

*FIG. 11*

Performing a set of X-ray attenuation
measurements, using different settings of
energy bin thresholds
S1

Obtaining information about material
composition
S2

Determining, for each X-ray attenuation
measurement, a value of at least one
performance metric
S3

Sweep of
varying
threshold
settings to
find
optimum of
performance
metric for
given
material
composition

Selecting a custom set of energy bin
thresholds
S4

Determining calibration data
S5

FIG. 12

## Various options for calibration data:

### OPTION 1

Calibration data coupling selected custom set of energy bin thresholds to information about material composition

### OPTION 2

Calibration data includes specific selected custom set of energy bin thresholds coupled to specific material composition

### OPTION 3

Calibration data includes specific selected custom set of energy bin thresholds coupled to a combination of material composition information and specific setting of scan parameters

### OPTION 4

Calibration data includes specific selected custom set of energy bin thresholds coupled to a combination of material composition information and specific object size

### OPTION 5

Calibration data includes specific selected custom set of energy bin thresholds coupled to a combination of material composition information, specific setting of scan parameters and specific object size

## FIG. 13

Obtaining information representative of
material composition — S11

Selecting custom set of energy bin
thresholds by using at least information
representative of material composition as
input to a look-up data structure or function — S12

Applying selected custom set of energy bin
thresholds as at least part of operational
settings of X-ray detector — S13

## FIG. 14

**INPUT**
- **material composition**, and optionally
- setting of scan parameters, and/or
- object size, and/or
- pixel or module

Look-up data structure or function

MATCH

**OUTPUT**
Custom set of energy bin thresholds

*FIG. 15*

For input to look-up table

Scan type

Transformation between scan type and material composition

Material composition

*FIG. 16*

FIG. 17

FIG. 18

Selection of energy thresholds

*FIG. 19*

Optimization of energy thresholds

Initial energy thresholds

Performance metric(s)

Modify thresholds

Stopping criteria?

Coupling of energy thresholds

Material composition

Object size/thickness

Charge - sharing

Count rates and pile-up

Arrangement
of detector
modules

Arrangement
of pixels in a
detector
module

FIG. 20

Begin

X-ray attenuation measurement with predefined energy thresholds

1. various materials (e.g., known combination compositions such as Al, PE and thickness), and optionally
2. various projection angles, rotation speeds, and/or
3. count rates (mA and kVp)

Calculate at least one of the following for the various X-ray attenuation measurements:

1. $SDNR^2$ measures the performance for detecting a feature
2. $SNR^2$ measures the capability of making a quantitative measurement of the composition of the object

Repeat X-ray attenuation measurements with different choices of thresholds to maximize at least one of the following:
1. $SDNR^2$
2. $SNR^2$

Select optimum thresholds for each combination of material composition, and optionally material path lengths, X-ray spectrum, count rate and/or pixel or module location and collect calibration data

End

# FIG. 21

FIG. 22

| kVp | mA | Rotation Speed | Material Composition | Material Thickness | Energy Thresholds |
|---|---|---|---|---|---|
| 80 | 100 | 0.2 | m1 | t1 | Thr set 1 |
| 80 | 100 | 0.2 | m1 | t2 | Thr set 2 |
| 80 | 100 | 0.2 | m2 | t1 | Thr set 3 |
| 80 | 100 | 0.2 | m2 | t2 | Thr set 4 |
| 80 | 100 | 0.2 | m1, m2 | t1, t2 | Thr set 5 |
| 80 | 100 | 0.2 | m2, m3, m4 | t2, t3, t4 | Thr set 6 |
| 80 | 100 | 0.2 | m1, m2, m3 | t1, t1, t1 | Thr set 7 |
| 90 | 110 | 0.15 | m1 | t1 | Thr set 8 |

# FIG. 23

Energy Threshold Settings:

Thr1 = [9 14 19 33 44 52 60 80 100]
Thr2 = [9 33 54 61 80]
Thr3 = [1 25 35 45 55 70 85 100]
Thr4 = [10 20 30]

*FIG. 24*

FIG. 25

FIG. 26

COMPUTER
PROGRAM
235

230

PROCESSOR
210

COMPUTER
PROGRAM
225

I/O
240

MEMORY
220

COMPUTER IMPLEMENTATION                    200

**EUROPEAN SEARCH REPORT**

Europäisches
Patentamt
European
Patent Office
Office européen
des brevets

**Application Number**

EP 25 17 9126

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RODESCH PIERRE-ANTOINE ET AL: "Photon-counting detector step-wedge calibration enabling water and iodine material decomposition", 20240513, vol. 19, no. 5, 13 May 2024 (2024-05-13), XP020500472, DOI: 10.1088/1748-0221/19/05/P05030 [retrieved on 2024-05-13] * 1, 2.1, 2.3, 2.7, 2.9, 3.1, 4; figures 1, 6, 10, 11; tables 2, 5 * | 1-14 | INV. G01T1/36 G01T7/00 |
| X | RICHTSMEIER DEVON ET AL: "Material decomposition with a prototype photon-counting detector CT system: expanding a stoichiometric dual-energy CT method via energy bin optimization and K-edge imaging", 20240219, vol. 69, no. 5, 19 February 2024 (2024-02-19), XP020490310, DOI: 10.1088/1361-6560/AD25C8 [retrieved on 2024-02-19] * 1, 2.7; table 2 * | 1-14 | TECHNICAL FIELDS SEARCHED (IPC) G01T |

-----

-----

The present search report has been drawn up for all claims

| Place of search | Date of completion of the search | Examiner |
|---|---|---|
| The Hague | 3 October 2025 | Johnstone, John |

EPO FORM 1503 03.82 (P4C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8183535 B

**Non-patent literature cited in the description**

- **ALVAREZ, MACOVSKI**. Energy-selective reconstructions in X-ray computerized tomography. *Phys Med Biol.*, 1976, vol. 21 (5), 733-744

- **ROESSL** ; **PROKSA**. K-edge imaging in X-ray computed tomography using multi-bin photon counting detectors. *Phys. Med. Biol.*, 2007, vol. 52, 4679-4696